(19) Europäisches Patentamt
European Patent Office
Office européen des brevets

(11) EP 3 278 737 A1

(12) **EUROPEAN PATENT APPLICATION**
published in accordance with Art. 153(4) EPC

(43) Date of publication:
**07.02.2018 Bulletin 2018/06**

(21) Application number: **15887766.2**

(22) Date of filing: **02.12.2015**

(51) Int Cl.:
***A61B 8/14*** *(2006.01)* ***A61B 8/12*** *(2006.01)*

(86) International application number:
**PCT/JP2015/083938**

(87) International publication number:
**WO 2016/157624 (06.10.2016 Gazette 2016/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.03.2015 JP 2015072726**

(71) Applicant: **OLYMPUS CORPORATION**
**Hachioji-shi**
**Tokyo 192-8507 (JP)**

(72) Inventor: **NAKATSUJI, Tomohiro**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

# (54) ULTRASONIC OBSERVATION APPARATUS, OPERATING METHOD OF ULTRASONIC OBSERVATION APPARATUS, AND OPERATING PROGRAM FOR ULTRASONIC OBSERVATION APPARATUS

(57) An ultrasound observation apparatus according to the present invention includes: a frequency analyzing unit that calculates a plurality of frequency spectra by analyzing a frequency of a signal generated based on an echo signal; a feature calculating unit that calculates feature of each of the plurality of frequency spectra, for each of three attenuation rate candidate values, calculates corrected features of the plurality of frequency spectra, and calculates a statistical dispersion of the corrected feature for each of the attenuation rate candidate values, then, generates a quadratic function based on the dispersion, and sets an attenuation rate candidate value for which the statistical dispersion in the quadratic function is minimum as an optimal attenuation rate; and a feature image data generating unit that generates feature image data displaying the corrected feature based on the optimal attenuation rate together with an ultrasound image.

FIG.8

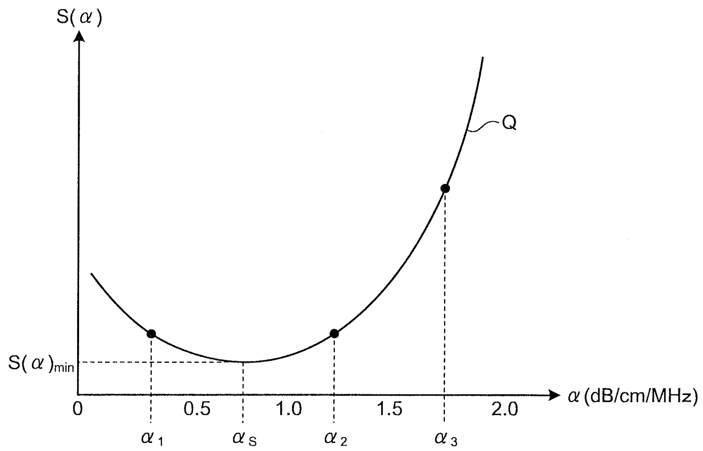


EP 3 278 737 A1

## Description

Field

The present invention relates to an ultrasound observation apparatus for observing tissues as an observation target by using ultrasound waves, a method for operating the ultrasound observation apparatus, and a program for operating the ultrasound observation apparatus.

Background

In order to observe the characteristics of body tissues or material as an observation target, there are cases where ultrasound waves are used. More specifically, by transmitting an ultrasound wave to an observation target and performing predetermined signal processing for an ultrasound echo reflected from the observation target, information relating to the characteristics of the observation target is acquired.

The intensity of an ultrasound wave attenuates when propagating through an observation target. Conventionally, a technology for determining the characteristics of the material of an observation target by using such attenuation is known (for example, see Patent Literature 1). According to such a technology, an electric signal corresponding to an ultrasound echo is transformed into an amplitude spectrum of the frequency domain, an attenuation amount is calculated by comparing the amplitude spectrum with a predetermined reference amplitude spectrum, and the attenuation amount is fitted to an attenuation model that depends on the characteristics of the material, whereby the characteristics of the material are determined. Citation List

Patent Literature

Patent Literature 1: JP-T 2008-545123

Summary

Technical Problem

In the technology disclosed in Patent Literature 1 described above, the reference amplitude spectrum has a same shape as that of the observation target, has an ultrasound wave speed that is equivalent to that of the observation target, and is set using a reference target (reference piece) of a material for which the attenuation of the ultrasound wave does not substantially occur. The method of determining the characteristics of an observation target by using a reference amplitude spectrum set in this way is effective in the case of a material having a regular structure but is difficult to apply in the case of a body tissue having an irregular structure.

In the technology disclosed in Patent Literature 1 described above, in order to determine the characteristics of a material, a plurality of different attenuation models are used according to the causes of the attenuation such as scattering and absorption, and accordingly, it is necessary to perform many calculation operations.

The present invention has been made in view of the foregoing, and an object of the invention is to provide an ultrasound observation apparatus capable of acquiring attenuation characteristics of an ultrasound wave suitable for an observation target through simple calculation and performing an observation using the attenuation characteristics, a method for operating the ultrasound observation apparatus, and a program for operating the ultrasound observation apparatus.

Solution to Problem

In order to solve the above described problem and achieve the object, an ultrasound observation apparatus according to the invention includes: a frequency analyzing unit configured to calculate a plurality of frequency spectra by analyzing a frequency of a signal generated based on an echo signal acquired by converting an ultrasound echo into an electric signal, the ultrasound echo being an ultrasound wave transmitted to an observation target and reflected from the observation target; a feature calculating unit configured to: calculate features of the plurality of frequency spectra; perform an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra; calculate a statistical dispersion of the corrected features for each of the at least three attenuation rate candidate values; generate a quadratic function based on the statistical dispersion; and set one of the at least three attenuation rate candidate values which gives a minimum statistical dispersion in the quadratic function, as an optimal attenuation rate; and a feature image data generating unit configured to generate feature image data for displaying the corrected features based on the optimal attenuation rate in association with visual information together with an ultrasound image generated from the echo signal.

In the ultrasound observation apparatus according to the invention, the feature calculating unit is configured to set the optimal attenuation rate by using data of a dynamic range wider than a dynamic range of data used by the feature image data generating unit.

In the ultrasound observation apparatus according to the invention, the feature calculating unit is configured to approximate each of the plurality of frequency spectra by an n-th order expression (n is a positive integer) to calculate the features.

In the ultrasound observation apparatus according to the invention, the feature calculating unit is configured to: approximate a predetermined frequency band of each of the plurality of frequency spectra by a linear expression; calculate, as the features, one or more of an intercept of the linear expression, a slope of the linear expression, and a mid-band fit that is a value of the linear expression in an intermediate frequency of the predetermined frequency band, the features including one of the slope and the mid-band fit; and set the optimal attenuation rate based on one of the slope and the mid-band fit.

In the ultrasound observation apparatus according to the invention, the feature calculating unit is configured to: set the optimal attenuation rate based on the slope if the slope is calculated as the features; and set the optimal attenuation rate based on the mid-band fit if the mid-band fit is calculated as the features.

In the ultrasound observation apparatus according to the invention, the feature calculating unit is configured to set the optimal attenuation rate for all frames of the ultrasound image.

In the ultrasound observation apparatus according to the invention, the feature calculating unit is configured to: set the optimal attenuation rate for every predetermined number of frames larger than one frame of the ultrasound image; and calculate the features of each of the plurality of frequency spectra for a frame for which the optimal attenuation rate is not set, by using the optimal attenuation rate that is set last before the frame.

In the ultrasound observation apparatus according to the invention, the feature calculating unit is configured to: calculate optimal attenuation rate correspondence values corresponding to the optimal attenuation rate for all frames of the ultrasound image; and set the optimal attenuation rate based on the optimal attenuation rate correspondence values calculated for a predetermined number of frames larger than one frame.

In the ultrasound observation apparatus according to the invention, the feature image data contains information on the optimal attenuation rate.

The ultrasound observation apparatus according to the invention further includes a display unit configured to display a feature image corresponding to the feature image data.

The ultrasound observation apparatus according to the invention further includes an input unit configured to receive an input for setting a target area for which the plurality of frequency spectra is calculated by the frequency analyzing unit. The frequency analyzing unit is configured to calculate the plurality of frequency spectra based on the ultrasound echo reflected from the target area.

A method for operating an ultrasound observation apparatus according to the invention includes: a frequency analyzing step of calculating a plurality of frequency spectra by analyzing a frequency of a signal generated based on an echo signal acquired by converting an ultrasound echo into an electric signal, the ultrasound echo being an ultrasound wave transmitted to an observation target and reflected from the observation target; a feature calculating step of, by a feature calculating unit: calculating features of the plurality of frequency spectra; performing an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra; calculating a statistical dispersion of the corrected features for each of the at least three attenuation rate candidate values; generating a quadratic function based on the statistical dispersion; and setting one of the at least three attenuation rate candidate values which gives a minimum statistical dispersion in the quadratic function, as an optimal attenuation rate; and a feature image data generating step of, by a feature image data generating unit, generating feature image data for displaying the corrected features based on the optimal attenuation rate in association with visual information together with an ultrasound image generated from the echo signal.

A program for operating an ultrasound observation apparatus according to the invention causes the ultrasound observation apparatus to execute: a frequency analyzing step of calculating a plurality of frequency spectra by analyzing a frequency of a signal generated based on an echo signal acquired by converting an ultrasound echo into an electric signal, the ultrasound echo being an ultrasound wave transmitted to an observation target and reflected from the observation target; a feature calculating step of, by a feature calculating unit: calculating features of the plurality of frequency spectra; performing an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra; calculating a statistical dispersion of the corrected features for each of the at least three attenuation rate candidate values; generating a quadratic function based on the statistical dispersion; and setting one of the at least three attenuation rate candidate values which gives a minimum statistical dispersion in the quadratic function, as an optimal attenuation rate; and a feature image data generating step of, by a feature image data generating unit, generating feature image data for displaying the corrected features based on the optimal attenuation rate in association with visual information together with an ultrasound image generated from the echo signal. Advantageous Effects of Invention

According to the present invention, it is possible to acquire attenuation characteristics of an ultrasound wave suitable for an observation target through simple calculation and to perform an observation using the attenuation characteristics.

Brief Description of Drawings

FIG. 1 is a block diagram illustrating the configuration of an ultrasound observation system including an ultrasound observation apparatus according to an embodiment of the present invention.
FIG. 2 is a graph illustrating a relation between a reception depth and an amplification factor in an amplification process performed by a signal amplifying unit of an ultrasound observation apparatus according to the embodiment of the present invention.
FIG. 3 is a graph illustrating a relation between a reception depth and an amplification factor in an amplification correcting process performed by an amplification correcting unit of an ultrasound observation apparatus according to the embodiment of the present invention.
FIG. 4 is a schematic diagram illustrating a data arrangement in one sound ray of an ultrasound signal.
FIG. 5 is a graph illustrating an example of a frequency spectrum calculated by a frequency analyzing unit of an ultrasound observation apparatus according to the embodiment of the present invention.
FIG. 6 is a graph illustrating a straight line having, as a parameter, a corrected feature obtained by an attenuation correcting unit of an ultrasound observation apparatus according to the embodiment of the present invention.
FIG. 7 is a graph illustrating an example of the distribution of corrected features obtained by performing attenuation correction based on two different attenuation rate candidate values for a same observation target.
FIG. 8 is a graph illustrating a relation between attenuation rate candidate values and the dispersion of corrected features obtained by performing the attenuation correction based on the attenuation rate candidate values.
FIG. 9 is a flowchart illustrating an overview of a process performed by an ultrasound observation apparatus according to the embodiment of the present invention.
FIG. 10 is a flowchart illustrating an overview of a process performed by a frequency analyzing unit of an ultrasound observation apparatus according to the embodiment of the present invention.
FIG. 11 is a schematic diagram illustrating an example of a feature image on a display device of an ultrasound observation system according to the embodiment of the present invention.

Description of Embodiments

Hereinafter, modes for carrying out the present invention (hereinafter, referred to as "embodiment(s)") will be described with reference to the attached drawings.

(Embodiments)

FIG. 1 is a block diagram illustrating the configuration of an ultrasound observation system including an ultrasound observation apparatus according to an embodiment of the present invention. An ultrasound observation system 1 illustrated in the drawing includes: an ultrasound endoscope 2 that transmits an ultrasound wave to a subject as an observation target and receives an ultrasound wave reflected from the subject; an ultrasound observation apparatus 3 that generates an ultrasound image based on an ultrasound signal acquired by the ultrasound endoscope 2; and a display device 4 that displays the ultrasound image generated by the ultrasound observation apparatus 3.

The ultrasound endoscope 2, in a tip end portion thereof, includes an ultrasound transducer 21 that converts an electric pulse signal received from the ultrasound observation apparatus 3 into an ultrasound pulse (acoustic pulse) and emits the ultrasound pulse to a subject and converts an ultrasound echo reflected from the subject into an electric echo signal represented using a voltage change and outputs the echo signal.

The ultrasound endoscope 2 generally includes an imaging optical system and an imaging device, is inserted into a digestive tract (esophagus, stomach, duodenum, or large intestine) or respiratory organs (trachea or a bronchial tube) of a subject, and is capable of imaging the digestive tract, the respiratory organs, and peripheral organs thereof (pancreas, gallbladder, a bile duct, a biliary tract, a lymph node, mediastinum organs, blood vessels, and the like). In addition, the ultrasound endoscope 2 includes a light guide that guides illumination light to be emitted to a subject at the time of imaging. This light guide has a tip end portion extending up to a tip end of a subject insertion portion of the ultrasound endoscope 2 and a base end portion connected to a light source device generating illumination light.

The ultrasound observation apparatus 3 includes: a transmitting and receiving unit 31 that is electrically connected to the ultrasound endoscope 2, transmits a transmission signal (pulse signal) configured by a high-voltage pulse to the ultrasound transducer 21 based on a predetermined waveform and transmission timing, receives an echo signal that is an electric reception signal from the ultrasound transducer 21, and generates and outputs data of a digital high frequency (radio frequency (RF)) signal (hereinafter, referred to as RF data); a signal processing unit 32 that generates digital B-mode reception data based on the RF data received from the transmitting and receiving unit 31; a computing unit 33 that performs a predetermined arithmetic operation for the RF data received from the transmitting and receiving unit 31; an image processing unit 34 that generates various kinds of image data; an input unit 35 that is realized by using a user interface such as a keyboard, a mouse, a touch panel, or the like and receives input of various kinds of information; a control unit 36 that controls the overall operation of the ultrasound observation system 1; and a storage unit 37 that stores various kinds of information necessary for the operation of the ultrasound observation apparatus 3.

The transmitting and receiving unit 31 includes a signal amplifying unit 311 that amplifies an echo signal. The signal amplifying unit 311 performs a sensitivity time control (STC) correction for amplification with a higher amplification factor for an echo signal having a large reception depth. FIG. 2 is a graph illustrating a relation between a reception depth and an amplification factor in an amplification process performed by the signal amplifying unit 311. A reception depth z illustrated in FIG. 2 is an amount calculated based on a time elapsing from a reception start time point of an ultrasound wave. As illustrated in FIG. 2, in a case where the reception depth z is smaller than a threshold $z_{th}$, an amplification factor $\beta$ (dB) is linearly increased from $\beta_0$ to $\beta_{th}$ (> $\beta_0$) in accordance with an increase in the reception depth z. On the other hand, in a case where the reception depth z is the threshold $z_{th}$ or more, the amplification factor P (dB) takes a constant value $\beta_{th}$. The value of the threshold $z_{th}$ is a value in which an ultrasound signal received from an observation target almost attenuates, and a noise is dominant. More generally, the amplification factor P may be monotonously increased according to an increase in the reception depth z in a case where the reception depth z is smaller than the threshold $z_{th}$. The relation illustrated in FIG. 2 is stored in the storage unit 37 in advance.

After performing a filtering process and the like for an echo signal amplified by the signal amplifying unit 311, the transmitting and receiving unit 31 generates RF data of the time domain by performing an A/D conversion and outputs the generated RF data to the signal processing unit 32 and the computing unit 33. In a case where the ultrasound endoscope 2 has a configuration for electronically scanning the ultrasound transducer 21 in which a plurality of elements are disposed in an array pattern, the transmitting and receiving unit 31 includes a multi-channel circuit used for beam synthesis corresponding to the plurality of elements.

The frequency band of a pulse signal transmitted by the transmitting and receiving unit 31 may be a broadband that almost covers the linear response frequency band of the electric acoustic conversion of a pulse signal into an ultrasound pulse in the ultrasound transducer 21. The frequency bands of various processes of an echo signal performed in the signal amplifying unit 311 may be a broadband that almost covers the linear response frequency band of the acoustic electric conversion of an ultrasound echo into an echo signal that is performed by the ultrasound transducer 21. Accordingly, when an approximation process of a frequency spectrum to be described later is performed, approximation having high accuracy can be performed.

The transmitting and receiving unit 31 also has a function for transmitting various control signals output by the control unit 36 to the ultrasound endoscope 2 and receiving various kinds of information including an identification ID from the ultrasound endoscope 2 and transmitting the received information to the control unit 36.

The signal processing unit 32 performs known processes such as band pass filtering, envelope detection, and a logarithmic conversion for RF data, thereby generating digital B-mode reception data. In the logarithmic conversion, a common logarithm of a quantity acquired by dividing RF data by a reference voltage $V_c$ is taken and is represented in a decibel value. The signal processing unit 32 outputs the generated B-mode reception data to the image processing unit 34. The signal processing unit 32 is realized by using a central processing unit (CPU), various arithmetic operation circuits, and the like.

The computing unit 33 includes: an amplification correcting unit 331 that performs an amplification correction such that the amplification factor P is constant for the RF data generated by the transmitting and receiving unit 31 regardless of the reception depth; a frequency analyzing unit 332 that calculates a frequency spectrum by performing a frequency analysis by performing a fast Fourier transform (FFT) for the RF data for which the amplification correction has been performed; and a feature calculating unit 333 that calculates a feature of the frequency spectrum. The computing unit 33 is realized by a central processing unit (CPU), various arithmetic operation circuits, and the like.

FIG. 3 is a graph illustrating a relation between a reception depth and an amplification factor in an amplification correcting process performed by the amplification correcting unit 331. As illustrated in FIG. 3, the amplification factor P (dB) in the amplification process performed by the amplification correcting unit 331 takes a maximum value $\beta_{th}$ - $\beta_0$ when the reception depth z is zero, linearly decreases until the reception depth z reaches the threshold $z_{th}$ from zero, and is zero when the reception depth z is the threshold $z_{th}$ or more. The amplification correcting unit 331 performs amplification correction on a digital RF signal by using the amplification factor determined in this way to offset the effect of an STC correction performed by the signal processing unit 32, which makes it possible to output a signal of a constant amplification factor $\beta_{th}$. Obviously, the relation between the reception depth z and the amplification factor P performed by the amplification correcting unit 331 differs depending on the relation between the reception depth and the amplification factor in the signal processing unit 32.

The reason for performing the amplification correction will be described. The STC correction is a correction process for excluding the influence of attenuation from the amplitude of an analog signal waveform by uniformly amplifying the amplitude of the analog signal waveform over the whole frequency band and amplifying the depth by using an amplification factor that is monotonously increased. For this reason, in a case where a B-mode image to be displayed is generated by converting the amplitude of an echo signal into the luminance and, in a case where a uniform tissue is scanned, by performing the STC correction, the luminance value becomes constant regardless of the depth. In other words, an effect of excluding the influence of attenuation from the luminance value of the B-mode image can be acquired.

On the other hand, in a case where an analysis result acquired by calculating the frequency spectrum of an ultrasound wave is used as in the embodiment, the influence of attenuation associated with the propagation of the ultrasound wave cannot be accurately excluded even by the STC correction. The reason for this is that, generally, while the attenuation amount is different according to the frequency (see Equation (1) to be described later), the amplification factor of the STC correction changes according to the distance and has no dependency on the frequency.

In order to address the situation described above, in other words, the situation that the influence of attenuation associated with the propagation of the ultrasound wave is not accurately excluded even by the STC correction if an analysis result acquired by calculating the frequency spectrum of an ultrasound wave is used, a method may be employed in which a reception signal for which the STC correction is performed is output when a B-mode image is generated, and, when an image that is based on the frequency spectrum is generated, new transmission other than transmission for generating the B-mode image is performed, and a reception signal for which the STC correction is not performed is output. However, in such a case, the frame rate of image data generated based on a reception signal may be decreased.

Thus, in the embodiment, in order to exclude the influence of the STC correction for a signal for which the STC correction is performed for a B-mode image while the frame rate of generated image data is maintained, the amplification factor is corrected by the amplification correcting unit 331.

The frequency analyzing unit 332 performs sampling of RF data (line data) of each sound ray for which an amplification correction is performed by the amplification correcting unit 331 at a predetermined time interval, thereby generating sample data. The frequency analyzing unit 332 performs an FFT process for a sample data group, thereby calculating a frequency spectrum at a plurality of positions (data positions) on the RF data.

FIG. 4 is a schematic diagram illustrating a data arrangement in one sound ray of an ultrasound signal. In a sound ray $SR_k$ illustrated in the drawing, a white rectangle or a black rectangle represents data at one sample point. In the sound ray $SR_k$, as data is positioned on a further right side, the data is sample data of a deeper position in a case where the position is measured from the ultrasound transducer 21 along the sound ray $SR_k$. The sound ray $SR_k$ is configured to be discrete at a time interval corresponding to a sampling frequency (for example, 50 MHz) of an A/D conversion performed by the transmitting and receiving unit 31. In FIG. 4, while a case is illustrated in which an eighth data position of the sound ray $SR_k$ of a number k is set as an initial value $Z^{(k)}{}_0$ in the direction of the reception depth z, the position of the initial value may be arbitrarily set. The result of the calculation acquired by the frequency analyzing unit 332 is acquired as a complex number and is stored in the storage unit 37.

A data group $F_j$ (j = 1, 2, ···, K) illustrated in Fig. 4 is a sample data group that is a target for an FFT process. Generally, in order to perform FFT, the number of data in a sample data group needs to be power of two. Hence, while a sample data group $F_j$ (j = 1, 2, ···, K - 1) is a normal data group having 16 (= $2^4$) data, a sample data group $F_K$ is an abnormal data group because the number of data is 12. In order to perform FFT on an abnormal data group, a process of generating a normal sample data group is performed by inserting zero data to cover the shortfall. This point will be described in detail when the process performed by the frequency analyzing unit 332 is described (see FIG. 9).

FIG. 5 is a graph illustrating an example of a frequency spectrum calculated by the frequency analyzing unit 332. The "frequency spectrum" represents a "frequency distribution of intensities for a certain reception depth z" acquired by performing FFT on a sample data group. The "intensity" described here, for example, represents a parameter of a voltage of an echo signal, power of an echo signal, sound pressure of an ultrasound echo, acoustic energy of an ultrasound echo, or the like, the amplitude or the time integration value of the parameter, or a combination thereof.

In FIG. 5, the horizontal axis is the frequency f. In addition, in FIG. 5, the vertical axis is a common logarithm (represented in decibels) $I = 10\log_{10}(I_0/I_c)$ of a quantity acquired by dividing the intensity $I_0$ by a reference intensity $I_c$ (constant). A straight line $L_{10}$ illustrated in FIG. 5 will be described later. In the embodiment, a curve or a straight line is configured by a set of discrete points.

In a frequency spectrum $C_1$ illustrated in FIG. 5, a lower limit frequency $f_L$ and an upper limit frequency $f_H$ of a frequency band used for the arithmetic operation performed thereafter are parameters that are determined based on the frequency band of the ultrasound transducer 21, the frequency band of a pulse signal transmitted by the transmitting and receiving unit 31, and the like. Hereinafter, as illustrated in FIG. 5, a frequency band set by the lower limit frequency $f_L$ and the upper limit frequency $f_H$ will be referred to as a "frequency band U".

Generally, in a case where an observation target is a body tissue, a frequency spectrum represents a different tendency in accordance with characteristics of body tissues scanned by an ultrasound wave. The reason for this is that the frequency spectrum has a correlation with the size, the number density, the acoustic impedance, and the like of a scattering body scattering an ultrasound wave. The "characteristics of body tissues" described here, for example, are a malignant tumor, a benign tumor, an endocrine tumor, a mucinous tumor, a normal tissue, a cyst, a vessel, and the like.

The feature calculating unit 333 calculates the feature of each of a plurality of frequency spectra, calculates corrected feature of each frequency spectrum by performing an attenuation correction for excluding the influence of attenuation of an ultrasound wave for feature (hereinafter, referred to as pre-correction feature) of each frequency spectrum for each of a plurality of attenuation rate candidate values giving different attenuation characteristics at a time when an ultrasound wave propagates through an observation target, and sets an attenuation rate that is optimal for the observation target among the plurality of attenuation rate candidate values by using the corrected feature.

The feature calculating unit 333 includes: an approximation unit 333a that calculates a pre-correction feature of a frequency spectrum by approximating the frequency spectrum by a straight line; an attenuation correcting unit 333b that calculates corrected feature by performing an attenuation correction based on each of a plurality of attenuation rate candidate values for the pre-correction feature calculated by the approximation unit 333a; and an optimal attenuation rate setting unit 333c that sets an optimal attenuation rate among the plurality of attenuation rate candidate values based on a statistical dispersion of the corrected feature calculated by the attenuation correcting unit 333b for all the frequency spectra.

The approximation unit 333a approximates a frequency spectrum by a linear expression (regression line) by performing a regression analysis on the frequency spectrum in a predetermined frequency band, thereby obtaining pre-correction features which define the linear expression. For example, in the case of the frequency spectrum $C_1$ illustrated in FIG. 5, the approximation unit 333a acquires a regression line $L_{10}$ by approximating the frequency spectrum $C_1$ by a linear expression by performing the regression analysis on a frequency band U. In other words, the approximation unit 333a calculates, as the pre-correction features, a slope $a_0$ and an intercept $b_0$ of the regression line $L_{10}$ and a mid-band fit $c_0 = a_0 f_M + b_0$ that is a value on the regression line of the center frequency $f_M = (f_L + f_H)/2$ of the frequency band U.

Among three pre-correction features, the slope $a_0$ has a correlation with the size of a scattering body of an ultrasound wave and, generally, the slope is considered to have a smaller value as the size of the scattering body is larger. The intercept $b_0$ has correlations with the size of a scattering body, a difference in the acoustic impedance, the number density (density) of the scattering body, and the like. More specifically, it is considered that the intercept $b_0$ has a larger value as the size of the scattering body is larger, has a larger value as the difference in the acoustic impedance is larger, and has a larger value as the number density of the scattering body is larger. The mid-band fit $c_0$ is an indirect parameter that is derived from the slope $a_0$ and the intercept $b_0$ and gives the intensity of the spectrum disposed at the center within an effective frequency band. For this reason, the mid-band fit $c_0$ is considered to have a correlation with the luminance of a B-mode image to some degree in addition to the size of the scattering body, the difference in the acoustic impedance, and the number density of the scattering body. The feature calculating unit 333 may approximate the frequency spectrum by a second-order polynomial or higher-order polynomial using regression analysis.

The correction performed by the attenuation correcting unit 333b will be described. Generally, the attenuation amount A(f, z) of an ultrasound wave is attenuation occurring while the ultrasound wave reciprocates between a reception depth 0 and a reception depth z and is defined as a change (a difference represented in decibel) in the intensity before and after the reciprocation. The attenuation amount A(f, z) is empirically known to be proportional to the frequency within a uniform tissue and is represented in the following Equation (1).

$$A(f, z) = 2\alpha z f \quad \cdots \quad (1)$$

Here, a proportion constant $\alpha$ is a quantity called an attenuation rate. In addition, z represents a reception depth of an ultrasound wave, and f represents a frequency. In a case where the observation target is a living body, a specific value of the attenuation rate $\alpha$ is determined according to a portion of the living body. The unit of the attenuation rate $\alpha$, for example, is dB/cm/MHz. In the embodiment, the attenuation correcting unit 333b, in order to set a most appropriate attenuation rate (optimal attenuation rate), performs an attenuation correction for each of a plurality of attenuation rate candidate values. The plurality of attenuation rate candidate values will be described in detail later with reference to FIG. 8.

The attenuation correcting unit 333b calculates corrected features a, b, and c by performing an attenuation correction on the pre-correction features (the slope $a_0$, the intercept $b_0$, and the mid-band fit $c_0$) extracted by the approximation unit 333a using Equations (2) to (4) represented below.

$$a = a_0 + 2\alpha z \cdots (2)$$

$$b = b_0 \cdots (3)$$

$$c = c_0 + A(f_M, z) = c_0 + 2\alpha z f_M (= a f_M + b) \cdots (4)$$

As is clear from Equations (2) and (4), the attenuation correcting unit 333b performs a correction having a larger correction amount as the reception depth z of the ultrasound wave is larger. According to Equation (3), a correction for the intercept is an identical transformation. The reason for this is that the intercept is a frequency component corresponding to a frequency 0 (Hz) and does not receive the influence of the attenuation.

FIG. 6 is a graph illustrating a straight line having corrected features a, b, and c obtained by the attenuation correcting unit 333b as parameters. The equation of the straight line $L_1$ is represented as below.

$$I = af + b = (a_0 + 2\alpha z) f + b_0 \cdots (5)$$

As is clear from the Equation (5), the straight line $L_1$ has a larger slope ($a > a_0$) than that of the straight line $L_{10}$ before the attenuation correction and has a same intercept ($b = b_0$) as that of the straight line $L_{10}$ before the attenuation correction.

The optimal attenuation rate setting unit 333c sets, as an optimal attenuation rate, an attenuation rate candidate value which gives a minimum statistical dispersion of the corrected feature calculated by the attenuation correcting unit 333b for each attenuation rate candidate value for all the frequency spectra. In the embodiment, as a quantity representing a statistical dispersion, a dispersion is applied. In this case, the optimal attenuation rate setting unit 333c sets, as then optimal attenuation rate, an attenuation rate candidate value which gives the minimum dispersion. Among the three corrected features a, b, and c, two pieces are independent. The corrected feature b does not depend on the attenuation rate. Accordingly, in a case where an optimal attenuation rate is set for the corrected features a and c, the optimal attenuation rate setting unit 333c may calculate the dispersion of one of the corrected features a and c.

However, the corrected feature used when the optimal attenuation rate is set by the optimal attenuation rate setting unit 333c is preferably a same type as that of the corrected feature used when feature image data is generated by a feature image data generating unit 342. In other words, it is preferable that the dispersion of the corrected feature a is applied in a case where the feature image data generating unit 342 generates feature image data by using a slope as the corrected feature, and the dispersion of the corrected feature c is applied in a case where the feature image data generating unit 342 generates feature image data by using a mid-band fit as the corrected feature. The reason for this is that Equation (1) giving the attenuation amount A(f, z) merely represents an ideal case, and practically, the following Equation (6) is appropriate.

$$A(f, z) = 2\alpha z f + 2\alpha_1 z \cdots (6)$$

$\alpha_1$ represented in the second term of the right-hand side represented in Equation (6) is a coefficient that represents a magnitude of a change in the signal intensity in proportion to the reception depth z of an ultrasound wave and is a coefficient that represents a change in the signal intensity occurring due to the non-uniformity of a tissue that is an observation target, a change in the number of channels at the time of beam synthesis, or the like. Since the second term of the right-hand side of Equation (6) is present, in a case where feature image data is generated using a mid-band fit as the corrected feature, the attenuation can be accurately corrected in a case where an optimal attenuation rate is set by using the dispersion of the corrected feature c (see Equation (4)). On the other hand, in a case where feature image data is generated using a slope that is a coefficient proportional to the frequency f, attenuation can be accurately corrected by excluding the influence of the second term of the right-hand side in a case where an optimal attenuation rate is set using the dispersion of the corrected feature a. For example, in a case where the unit of the attenuation rate $\alpha$ is dB/cm/MHz, the unit of the coefficient $\alpha_1$ is dB/cm.

Here, the reason why an optimal attenuation rate can be set based on the statistical dispersion will be described.

In a case where an optimal attenuation rate is applied to an observation target, it is considered that the feature converges to a value that is unique to the observation target regardless of a distance between the observation target and the ultrasound transducer 21, and a statistical dispersion is decreased. On the other hand, in a case where an attenuation rate candidate value that is not appropriate for the observation target is set as an optimal attenuation rate, the attenuation correction is excessive or insufficient, and accordingly, it is considered that a deviation occurs in the feature in accordance with a distance to the ultrasound transducer 21, and the feature is statistically irregular. Accordingly, an attenuation rate candidate value which gives the smallest statistical dispersion can be regarded as an optimal attenuation rate for the observation target.

FIG. 7 is a graph illustrating an example of the distribution of corrected features obtained by performing the attenuation correction based on two different attenuation rate candidate values for a same observation target. In FIG. 7, the horizontal axis is the corrected feature, and the vertical axis is the frequency. Two distribution curves $N_1$ and $N_2$ illustrated in FIG. 7 are the same as a total sum of frequencies. In the case illustrated in FIG. 7, the distribution curve $N_1$ has a statistical dispersion of the feature smaller than that of the distribution curve $N_2$ (smaller dispersion) and forms a shape having a peak steeper than that of the distribution curve $N_2$. Thus, in a case where an optimal attenuation rate is set from two attenuation rate candidate values corresponding to these two distribution curves $N_1$ and $N_2$, the optimal attenuation rate setting unit 333c sets an attenuation rate candidate value corresponding to the distribution curve $N_1$ as an optimal attenuation rate.

Generally, it is known that, for attenuation rate candidate values and the dispersion that is a statistical dispersion of the corrected feature calculated for each attenuation rate candidate value, one quadratic function is determined for each frame. In the embodiment, the optimal attenuation rate setting unit 333c acquires a minimal value (extreme value) in the quadratic function generated based on the dispersion of corrected features c obtained by performing the attenuation correction based on a plurality of attenuation rate candidate values (three attenuation rate candidate values in the embodiment) and sets an attenuation rate candidate value corresponding to the extreme value as an optimal attenuation rate. An optimal attenuation rate is set based on the extreme value by the fact that a true value of an attenuation rate is identical to an attenuation rate $\alpha$ which gives a minimal dispersion if an observation target is uniform. While four or more attenuation rate candidate values may be set, from the viewpoint of decreasing the load according to the arithmetic operation process, three attenuation rate candidate values are preferable.

In the embodiment, three attenuation rate candidate values (attenuation rate candidate values $\alpha_1$, $\alpha_2$, and $\alpha_3$) are stored in the storage unit 37 in advance, and the optimal attenuation rate setting unit 333c sets an optimal attenuation rate by using these three attenuation rate candidate values. The attenuation rate candidate values $\alpha_1$, $\alpha_2$, and $\alpha_3$ are values of 0.0 or more, and, in a case where the observation target is a body tissue, the attenuation rate of the body tissues is generally near 0.6, and accordingly, it is preferable that a smallest attenuation rate candidate value among the three attenuation rate candidate values is 0.6 or less, and a largest value thereof is 0.6 or more.

FIG. 8 is a graph illustrating a relation between attenuation rate candidate values $\alpha_1$, $\alpha_2$, and $\alpha_3$ and a quadratic function Q generated based on dispersions $S(\alpha_1)$, $S(\alpha_2)$, and $S(\alpha_3)$ of corrected features obtained by performing the attenuation correction based on the attenuation rate candidate values $\alpha_1$, $\alpha_2$, and $\alpha_3$. The optimal attenuation rate setting unit 333c acquires dispersions $S(\alpha_1)$, $S(\alpha_2)$, and $S(\alpha_3)$ of the corrected features c obtained by performing the attenuation correction based on the preset attenuation rate candidate values $\alpha_1$, $\alpha_2$, and $\alpha_3$ and generates a quadratic function Q passing through the acquired dispersions $S(\alpha_1)$, $S(\alpha_2)$, $S(\alpha_3)$. The quadratic function acquired at this time is a function that is convex downward. The optimal attenuation rate setting unit 333c acquires an extreme value of the generated quadratic function Q and sets an attenuation rate candidate value $\alpha_s$ corresponding to the extreme value as an optimal attenuation rate.

Here, in RF data of a same frame, the reason why the dispersions that are based on a plurality of attenuation rate candidate values are present on a same quadratic function, and the quadratic function is convex downward will be described. In description presented below, the dispersion of corrected feature c (mid-band fit) will be described as an example. When the dispersion of the corrected feature c is denoted by $V_c(\alpha)$, the following Equation (7) is derived from Equation (4) described above. In Equation (7), i is a subscript used for identifying a sample point, and dispersion $V_c(\alpha)$ is calculated by acquiring a sum of the square of a difference between each corrected feature $c_i$ and an arithmetic mean of corrected feature.

$$V_c(\alpha) = \Sigma_i (c_i - \bar{c})^2 \quad \cdots \quad (7)$$

From Equation (7) described above, the following Equation (8) is derived. In the following Equation (8), a reciprocation distance is L (L = 2z).

$$V_c(\alpha) = \Sigma_i\{(c_i + \alpha \cdot f_m \cdot L_i) - (\overline{c} + \alpha \cdot f_m \cdot \overline{L})\}^2$$
$$= \Sigma_i\{c_i - ci - \overline{c} + d + \alpha \cdot f_m \cdot (L_i - ave(L_i))\}^2$$
$$= \alpha^2 \cdot f_m^2 \cdot \Sigma_i(L_i - \overline{L})^2 + 2 \cdot f_m \cdot \alpha \cdot \Sigma_i(c_i - \overline{c}) \cdot (L_i - \overline{L}) + \Sigma_i(c_i - \overline{c})^2$$
$$= \left\{f_m^2 \cdot \Sigma_k(L_k - \overline{L})^2\right\} \cdot \left[\alpha^2 + 2 \cdot \alpha \cdot \Sigma_i(c_i - \overline{c}) \cdot (L_i - \overline{L}) / \left\{f_m \cdot \Sigma_j(L_j - \overline{L})^2\right\}\right] + \Sigma_i(c_i - \overline{c})^2$$
$$= \left\{f_m^2 \cdot \Sigma_k(L_k - \overline{L})^2\right\} \cdot \left[\alpha + (1/f_m) \cdot \left\{\Sigma_i(c_i - \overline{c}) \cdot (L_i - \overline{L}) / \Sigma_j(L_j - \overline{L})^2\right\}\right]^2 + \left[\left\{\Sigma_i(c_i - \overline{c})^2\right\} \cdot \left\{\Sigma_j(L_j - \overline{L})^2\right\} - \left\{\Sigma_i(c_i - \overline{c}) \cdot (L_i - \overline{L})\right\}^2\right] / \Sigma_k(L_k - \overline{L})^2 \quad \cdots (8)$$

In Equation (8), the coefficient of $\alpha^2$ in $V_c(\alpha)$ has a positive value. Thus, $V_c(\alpha)$ is a quadratic function that is convex downward.

For the dispersion of corrected feature a, when the dispersion of the corrected feature a is denoted by $V_a(\alpha)$, the following Equation (9) is derived from Equation (2) described above.

$$V_a(\alpha) = \Sigma_i(a_i - \overline{a})^2 \quad \cdots (9)$$

From Equation (9) described above, by performing calculation similar to the dispersion $V_c(\alpha)$ described above, the following Equation (10) is derived.

$$V_a(\alpha) = \Sigma_k(L_k - \overline{L})^2 \cdot \left\{\alpha + \Sigma_i(a_i - \overline{a}) \cdot (L_i - \overline{L}) / \Sigma_j(L_j - \overline{L})^2\right\}^2 + \left[\left\{\Sigma_i(a_i - \overline{a})^2\right\} \cdot \left\{\Sigma_j(L_j - \overline{L})^2\right\} - \left\{\Sigma_i(a_I - \overline{a}) \cdot (L_i - \overline{L})\right\}^2\right] / \Sigma_k(L_k - \overline{L})^2 \quad \cdots (10)$$

In Equation (10), the coefficient of $\alpha_2$ in $V_a(\alpha)$ has a positive value. Thus, also for $V_a(\alpha)$, a quadratic function that is convex downward is acquired.

The image processing unit 34 includes: a B-mode image data generating unit 341 that generates a B-mode image data that is an ultrasound image converting the amplitude of an echo signal into luminance and displaying the luminance; and a feature image data generating unit 342 that generates feature image data displaying feature that is based on an optimal attenuation rate set by the optimal attenuation rate setting unit 333c in association with visual information together with the B-mode image.

The B-mode image data generating unit 341, for B-mode reception data received from the signal processing unit 32, performs signal processing using known technologies such as gain processing and contrast processing and performs data interpolation according to a data step width determined based on the display range of an image in the display device 4, and the like, thereby generating a B-mode image data. The B-mode image is a gray scale image in which the values of R (red), G (green), and B (blue), which are variables in a case where a RGB color system is employed as a color space, match each other.

The B-mode image data generating unit 341, after performing a coordinate conversion of rearrangement for the B-mode reception data transmitted from the signal processing unit 32 such that the scanning range can be correctly spatially represented, performs an interpolation process for the B-mode reception data, and fills a gap between the B-mode reception data, thereby generating B-mode image data. The B-mode image data generating unit 341 outputs the generated B-mode image data to the feature image data generating unit 342.

The feature image data generating unit 342 superimposes visual information relating to the feature calculated by the feature calculating unit 333 on each pixel of an image of B-mode image data, thereby generating feature image data. The feature image data generating unit 342, for example, assigns visual information corresponding to feature of a frequency spectrum calculated from one sample data group $F_j$ (here, j = 1, 2, ···, K) to a pixel area corresponding to a data amount of the sample data group $F_j$ illustrated in FIG. 4. The feature image data generating unit 342, for example, associates hue as visual information with one of the slope, intercept, and the mid-band fit described above, thereby generating a feature image. The feature image data generating unit 342 may generate feature image data by associating hue with one of two features selected from the slope, the intercept, and the mid-band fit and associating shading with each other. As the visual information relating to the feature, for example, there are hue, saturation, brightness, a luminance value, and variables of a color space configuring a predetermined display color system such as R (red), G (green), and B (blue).

The control unit 36 is realized by a central processing unit (CPU) having an arithmetic operation and control function and various arithmetic operation circuits, and the like. The control unit 36 reads information stored by the storage unit 37 from the storage unit 37 and performs various arithmetic operation processes relating to an operation method of the ultrasound observation apparatus 3, thereby performs an overall control of the ultrasound observation apparatus 3. The control unit 36 may be configured by using a CPU and the like that are common to the signal processing unit 32 and the computing unit 33.

The storage unit 37 includes a feature information storing unit 371 that stores the attenuation rate candidate values $\alpha_1$, $\alpha_2$, and $\alpha_3$, a plurality of features calculated in accordance with attenuation rate candidate values by the attenuation correcting unit 333b for each frequency spectrum, and a dispersion giving a statistical dispersion of the plurality of features in association with the attenuation rate candidate values.

In addition to the information described above, the storage unit 37 stores, for example information (the relation between the amplification factor and the reception depth illustrated in Fig. 2) required for an amplification process, information (the relation between the amplification factor and the reception depth illustrated in FIG. 3) required for an amplification correcting process, information (see Equation (1)) required for the attenuation correcting process, and information of a window function (such as Hamming, Hanning, and Blackman) required for a frequency analyzing process.

The storage unit 37 further stores various programs including an operation program for performing the operation method of the ultrasound observation apparatus 3. The operation program may be recorded on a computer-readable recoding medium such as a hard disk, a flash memory, a CD-ROM, a DVD-ROM, or a flexible disk and be widely distributed. The various programs described above may be acquired by being downloaded through a communication network. The communication network described here, for example, is realized by an existing public circuit network, a local area network (LAN), a wide area network (WAN), or the like and may be either a wired network or a wireless network.

The storage unit 37 having the configuration described above is realized by a read only memory (ROM) in which various programs are installed in advance, a random access memory (RAM) storing arithmetic operation parameters and data of each process.

In the embodiment, in the attenuation correcting unit 333b, it is preferable that the dynamic range of the corrected feature is set in accordance with a range that can be set in the gain processing, the contrast processing, and the like performed by the image processing unit 34. More specifically, the attenuation rate candidate values $\alpha_1$, $\alpha_2$, and $\alpha_3$ are three values set between 0.0 and 2.0 such that the feature displayed as a feature image is within a dynamic range relating to display according to a range that can be set in the gain process, the contrast process, and the like described above. For example, in a case where the attenuation rate candidate value is larger than 2.0, there are cases where the calculated corrected feature exceeds the dynamic range and cannot be maintained as corrected feature. Accordingly, the values of the statistical dispersions become different, and, for example, a function that is generated based on the dispersions is not a quadratic function, and the attenuation rate cannot be set to an optimal value. In other words, the attenuation rate candidate values $\alpha_1$, $\alpha_2$, and $\alpha_3$ relating to the embodiment are values set in the range of 0.0 to 2.0, and it is preferable that a smallest attenuation rate candidate value among the three attenuation rate candidate values is 0.6 or less, and a largest value thereof is 0.6 or more. Particularly, if the observation target is body tissues and a unique point system is used, it is more preferable that three attenuation rate candidate values are respectively set to be around 0.6 in order to suppress a decrease in the calculation precision due to clip.

FIG. 9 is a flowchart illustrating an overview of a process performed by the ultrasound observation apparatus 3 having the configuration described above. First, the ultrasound observation apparatus 3 receives an echo signal as a result of the measurement of an observation target that is performed by the ultrasound transducer 21 from the ultrasound endoscope 2 (Step S1).

The signal amplifying unit 311 that has received the echo signal from the ultrasound transducer 21 amplifies the echo signal (Step S2). Here, the signal amplifying unit 311, for example, amplifies (STC correction) of the echo signal based on the relation between the amplification factor and the reception depth illustrated in FIG. 2.

Subsequently, the B-mode image data generating unit 341 generates B-mode image data by using the echo signal amplified by the signal amplifying unit 311 and outputs the generated B-mode image data to the display device 4 (Step S3). The display device 4 that has received the B-mode image data displays a B-mode image corresponding to the B-mode image data (Step S4).

The amplification correcting unit 331 performs an amplification correction having a constant amplification factor regardless of the reception depth for a signal output from the transmitting and receiving unit 31 (Step S5). Here, the amplification correcting unit 331, for example, performs an amplification correction such that the relation between the amplification factor and the reception depth illustrated in FIG. 3 is satisfied.

After the amplification correction, the frequency analyzing unit 332 calculates a frequency spectrum for all the sample data groups within a focused area that is an area for which feature image data is generated by performing a frequency analysis through the FFT operation (Step S6: frequency analyzing step). FIG. 10 is a flowchart illustrating an overview of a process performed by the frequency analyzing unit 332 in Step S6. Hereinafter, the frequency analyzing process will be described in detail with reference to the flowchart illustrated in FIG. 10.

First, the frequency analyzing unit 332 sets a counter k used for identifying a sound ray that is an analysis target to $k_0$ (Step S21).

Subsequently, the frequency analyzing unit 332 sets an initial value $Z^{(k)}_0$ of the data position (corresponding to the reception depth) $Z^{(k)}$ representing a series of data groups (sample data groups) acquired for the FFT operation (Step S22). For example, FIG. 4, as described above, illustrates a case where an eighth data position of the sound ray $SR_k$ is set as the initial value $Z^{(k)}_0$.

Thereafter, the frequency analyzing unit 332 acquires a sample data group (Step S23) and applies a window function stored in the storage unit 37 to the acquired sample data group (Step S24). In this way, by applying the window function to the sample data group, the sample data group is not discontinuous at the boundary, and the occurrence of an artifact can be prevented.

Subsequently, the frequency analyzing unit 332 determines whether or not the sample data group of a data position $Z^{(k)}$ is a normal data group (Step S25). As described with reference to FIG. 4, a sample data group needs to have the number of data that is power of two. Hereinafter, the number of data of a normal sample data group will be $2^n$ (n is a positive integer). In the embodiment, the data position $Z^{(k)}$ is set to be the center of a sample data group to which $Z^{(k)}$ belongs as possibly as can. More specifically, since the number of data of the sample data group is $2^n$, $Z^{(k)}$ is set to a $2^n/2$ (= $2^{n-1}$)-th position close to the center of the sample data group. In such a case, a sample data group being normal represents that $2^{n-1}$ - 1 (= N) pieces of data are present on the front side of the data position $Z^{(k)}$, and $2^{n-1}$ (= M) pieces of data are present on the rear side of the data position $Z^{(k)}$. In the case illustrated in FIG. 4, the sample data groups $F_j$ (j = 1, 2, ···, K - 1) are normal altogether. FIG. 4 illustrates a case where n = 4 (N = 7 and M = 8).

As a result of the determination acquired in Step S25, in a case where the sample data group of the data position $Z^{(k)}$ is normal (Step S25: Yes), the frequency analyzing unit 332 proceeds to Step S27 to be described later.

As a result of the determination acquired in Step S25, in a case where the sample data group of the data position $Z^{(k)}$ is not normal (Step S25: No), the frequency analyzing unit 332 generates a normal sample data group by inserting zero data to cover the shortfall (Step S26). A window function is applied to a sample data group (for example, a sample data group $F_K$ illustrated in FIG. 4) determined not to be normal in Step S25 before the insertion of zero data. For this reason, even when zero data is inserted to the sample data group, discontinuity of data does not occur. After Step S26, the frequency analyzing unit 332 proceeds to Step S27 to be described later.

In Step S27, the frequency analyzing unit 332 acquires a frequency spectrum that is a frequency distribution of amplitudes by performing the FFT by using the sample data group (Step S27). The frequency spectrum $C_1$ illustrated in FIG. 5 is an example of the frequency spectrum acquired as a result of Step S27.

Subsequently, the frequency analyzing unit 332 changes the data position $Z^{(k)}$ by a step width D (Step S28). The step width D is assumed to be stored in the storage unit 37 in advance. FIG. 4 illustrates a case where D = 15. While the step width D preferably coincides with a data step width used when the B-mode image data generating unit 341 generates a B-mode image data, in a case where the amount of calculation performed by the frequency analyzing unit 332 is desired to be decreased, a value lager than the data step width may be set as the step width D.

Thereafter, the frequency analyzing unit 332 determines whether or not the data position $Z^{(k)}$ is larger than a maximum value $Z^{(k)}_{max}$ in the sound ray $SR_k$ (Step S29). In a case where the data position $Z^{(k)}$ is larger than the maximum value $Z^{(k)}_{max}$ (Step S29: Yes), the frequency analyzing unit 332 increases the counter k by one (Step S30). This represents that the process proceeds to a next sound ray. On the other hand, in a case where the data position $Z^{(k)}$ is the maximum value $Z^{(k)}_{max}$ or less (Step S29: No), the frequency analyzing unit 332 causes the process to be returned to Step S23. In this way, the frequency analyzing unit 332 performs the FFT on $[(Z^{(k)}_{max} - Z^{(k)}_0 + 1)/D + 1]$ sample data groups for the sound ray $SR_k$. Here, [X] represents a maximum integer not exceeding X.

After Step S30, the frequency analyzing unit 332 determines whether or not the counter k is larger than the maximum value $k_{max}$ (Step S31). In a case where the counter k is larger than the maximum value $k_{max}$ (Step S31: Yes), the frequency analyzing unit 332 ends a series of frequency analyzing processes. On the other hand, in a case where the counter k is the maximum value $k_{max}$ or less (Step S31: No), the frequency analyzing unit 332 causes the process to be returned to Step S22. This maximum value $k_{max}$ is a value that is arbitrarily directed and input through the input unit 35 by a user such as an operator or a value that is set in the storage unit 37 in advance.

In this way, the frequency analyzing unit 332 performs FFT multiple times on each of ($k_{max}$ - $k_0$ + 1) sound rays within the analysis target area. The results of the FFT are stored in the storage unit 37 together with the reception depth and the reception direction.

In the description presented above, while the frequency analyzing process is performed only within the set focused area, the frequency analyzing unit 332 may be configured to perform the frequency analyzing process for all the areas from which an ultrasound signal is received.

Following the frequency analyzing process of Step S6 described above, the feature calculating unit 333 calculates pre-correction features of each of a plurality of frequency spectra, for each of a plurality of attenuation rate candidate values giving different attenuation characteristics when an ultrasound wave propagates through an observation target, calculates corrected feature of each frequency spectrum by performing an attenuation correction excluding the influence of attenuation of an ultrasound wave for the pre-correction features of each frequency spectrum, calculates a dispersion of each attenuation rate candidate value by using the corrected features, and sets an optimal attenuation rate for the observation target by generating a quadratic function representing the relation between the attenuation rate candidate value and the dispersion and acquiring an extreme value (Steps S7 to S12: feature calculating step). Hereinafter, the process of Steps S7 to S12 will be described in detail.

In Step S7, the approximation unit 333a performs a regression analysis of each of the plurality of frequency spectra calculated by the frequency analyzing unit 332, thereby calculating a pre-correction feature that corresponds to each frequency spectrum of a divided area as the attenuation rate setting target (Step S7). More specifically, the approximation unit 333a performs regression analysis on each frequency spectrum to approximate each frequency spectrum by a linear expression, and obtain a slope $a_0$, an intercept $b_0$ and a mid-band fit $c_0$, as the pre-correction feature. For example, the straight line $L_{10}$ illustrated in FIG. 5 is a regression line obtained by approximating the frequency spectrum $C_1$ in the frequency band U using regression analysis performed by the approximation unit 333a.

Thereafter, the optimal attenuation rate setting unit 333c sets values of attenuation rate candidate values applied when an attenuation correction to be described later is performed to predetermined set values $\alpha_1$, $\alpha_2$, and $\alpha_3$. It may be configured such that the values of the set values $\alpha_1$, $\alpha_2$, and $\alpha_3$ are stored in the feature information storing unit 371 in advance, and the optimal attenuation rate setting unit 333c refers to the feature information storing unit 371.

Subsequently, the attenuation correcting unit 333b performs attenuation correction on the pre-correction feature obtained by the approximation unit 333a by approximating each frequency spectrum, using the set values $\alpha_1$, $\alpha_2$, and $\alpha_3$ as attenuation rate candidate values to calculate corrected features, and stores the calculated corrected features in the feature information storing unit 371 together with the set values $\alpha_1$, $\alpha_2$, and $\alpha_3$ (Step S8). A straight line $L_1$ illustrated in FIG. 6 is an example of a straight line acquired by the attenuation correcting unit 333b performing the attenuation correcting process.

In Step S8, the attenuation correcting unit 333b calculates the corrected features by substituting the reception depth z in Equations (2) and (4) with the data position $Z = (f_{sp}/2v_s)Dn$ acquired by using a data arrangement of sound rays of an ultrasound signal. Here, $f_{sp}$ represents the sampling frequency of data, $v_s$ represents the speed of sound, D represents a data step width, and n represents the number of data steps from first data of a sound ray up to the data position of a sample data group that is a processing target. For example, when the sampling frequency $f_{sp}$ of data is 50 MHz, the speed of sound $v_s$ is 1,530 m/sec, and the step width D is 15 by employing the data arrangement illustrated in FIG. 6, $Z = 0.2295 \cdot n$ (mm).

The optimal attenuation rate setting unit 333c calculates the dispersion of representative corrected feature among a plurality of corrected features acquired by the attenuation correcting unit 333b performing an attenuation correction for each frequency spectrum and stores the calculated dispersions in the feature information storing unit 371 in association with the set values $\alpha_1$, $\alpha_2$, and $\alpha_3$ (Step S9). In a case where the corrected feature is a slope a and a mid-band fit c, like the case illustrated in FIG. 8 described above, the optimal attenuation rate setting unit 333c, for example, calculates the dispersion of the corrected feature c. In Step S19, it is preferable that the optimal attenuation rate setting unit 333c applies the dispersion of the feature a in a case where the feature image data generating unit 342 generates feature image data by using the slope and applies the dispersion of the corrected feature c in a case where feature image data is generated by using the mid-band fit.

Thereafter, the optimal attenuation rate setting unit 333c generates a quadratic function based on the dispersion of the corrected features c after the attenuation correction is performed based on the set values $\alpha_1$, $\alpha_2$, and $\alpha_3$ (Step S10). The optimal attenuation rate setting unit 333c acquires an extreme value of the generated quadratic function (Step S11) and sets, as an optimal attenuation rate, an attenuation rate candidate value corresponding to the extreme value (Step S12).

As illustrated in FIG. 8, in a case where the dispersion takes an extreme value $S(\alpha)_{min}$ when the attenuation rate candidate value $\alpha_s$ is 0.65 (dB/cm/MHz), the optimal attenuation rate setting unit 333c sets $\alpha = 0.65$ (dB/cm/MHz) as an optimal attenuation rate.

The feature image data generating unit 342 superimposes visual information (for example, hue) associated with the corrected feature that is based on the optimal attenuation rate specified in Step S12 on each pixel in the B-mode image data generated by the B-mode image data generating unit 341 and adds information of the optimal attenuation rate, thereby generating feature image data (Step S13: feature image data generating step).

Thereafter, the display device 4, under the control of the control unit 36, displays a feature image corresponding to the feature image data generated by the feature image data generating unit 342 (Step S14). FIG. 11 is a schematic diagram illustrating an example of display of a feature image in the display device 4. A feature image 201 illustrated in the drawing includes: a superimposed image display section 202 displaying an image in which visual information relating to feature is superimposed on a B-mode image; and an information display section 203 displaying identification information of an observation target and information of an attenuation rate candidate value set as the optimal attenuation rate. In the information display section 203, the information of the feature, the information of the approximation equation, the information of a quadratic function generated based on the dispersion of the corrected features obtained by performing the attenuation correction based on the set values $\alpha_1$, $\alpha_2$, and $\alpha_3$, image information of a gain, contrast, and the like may be further displayed. A B-mode image corresponding to a feature image may be displayed so as to be aligned with a feature image. Furthermore, the input unit 35 may be configured to receive a direction signal representing where or not the information of the attenuation rate candidate value is displayed.

In the series of processes (Steps S1 to S14) described above, the process of Step S4 and the process of Steps S5 to S12 may be configured to be performed in parallel.

According to the embodiment of the present invention described above, each corrected feature is calculated by performing an attenuation correction by using three set values (set values $\alpha_1$, $\alpha_2$, and $\alpha_3$) set in advance as attenuation rate candidate values, an extreme value of a quadratic function generated based on the dispersion of the corrected feature is acquired, and an attenuation rate candidate value corresponding to the extreme value is set as an optimal attenuation rate. Accordingly, the attenuation characteristics of an ultrasound wave that are appropriate for an observation target can be acquired at a high speed through simple calculation, and an observation using the attenuation characteristics can be performed.

In addition, according to the embodiment, an optimal partial attenuation rate is set based on a statistical dispersion of corrected feature acquired by performing an attenuation correction of each frequency spectrum, and accordingly, the amount of calculation can be smaller than that of a conventional case where fitting with a plurality of attenuation models is performed.

Furthermore, according to the embodiment, even if an attenuation rate that is appropriate for an observation target is unknown, it is possible to set an optimal attenuation rate.

In addition, according to the embodiment, by configuring the set values $\alpha_1$, $\alpha_2$, and $\alpha_3$ as the attenuation rate candidate values to have values of 0.0 or more, configuring a smallest attenuation rate among the three attenuation rate candidate values to be 0.6 or less, and configuring a largest value thereof to be 0.6 or more, the calculation accuracy of the optimal attenuation rate acquired when a body tissue generally having an attenuation rate near 0.6 is set as an observation target can be improved.

Furthermore, according to the embodiment, an attenuation rate corresponding to an extreme value of the quadratic function is set as the optimal attenuation rate. Accordingly, a numerical value having digits more than the attenuation rate candidate values set in advance can be set as the attenuation rate, whereby the calculation accuracy of the optimal attenuation rate can be improved.

In addition, according to the embodiment, by setting the attenuation rate candidate values $\alpha_1$, $\alpha_2$, $\alpha_3$ to be in the range of 0.0 to 2.0 such that feature displayed as a feature image is within the dynamic range, the calculation accuracy of the optimal attenuation rate acquired when a body tissue is set as an observation target can be improved.

Furthermore, according to the embodiment, by further displaying the information on a quadratic function generated based on the dispersion of corrected features obtained by performing the attenuation correction based on the set values $\alpha_1$, $\alpha_2$, $\alpha_3$ on the information display section 203, an error or the like occurring in the generation of the quadratic function can be checked and acquired by a user.

(Modified Example of Embodiments)

Next, a modified example of the embodiment of the present invention will be described. In the modified example, the optimal attenuation rate setting unit 333c sets an optimal attenuation rate in a dynamic range wider than a dynamic range at the time of displaying a feature image.

More specifically, when the display dynamic range of an image generated by the feature image data generating unit 342 is 70 dB, the feature calculating unit 333 performs an attenuation calculation process with a dynamic range (for example, 100 dB) larger than this dynamic range (70 dB). For example, while the feature image data generating unit 342 uses a fixed-point system of eight bits, the feature calculating unit 333 performs the attenuation calculating process including the calculation of feature to the setting of an optimal attenuation rate by using a floating-point system of 32 bits.

According to the modified example, compared to the attenuation calculating process using a fixed-point system, the calculation accuracy can be improved. By performing the generation of a quadratic function that is based on the dispersion from the calculation of the pre-correction feature with further higher accuracy, an optimal attenuation rate can be calculated with high accuracy.

Although the modes carrying out the present invention has been described, the present invention is not limited only by the embodiments described above. For example, the optimal attenuation rate setting unit 333c may calculate each optimal attenuation rate correspondence value corresponding to an optimal attenuation rate for all the frames of an ultrasound image and set a mean value, a median value, or a maximum frequency of a predetermined number of optimal attenuation rate correspondence values including an optimal attenuation rate correspondence value of a latest frame as an optimal attenuation rate. In such a case, a change in the optimal attenuation rate is smaller than that of a case where an optimal attenuation rate is set in each frame, and accordingly, the value thereof can be stabilized.

The optimal attenuation rate setting unit 333c may set an optimal attenuation rate at a predetermined frame interval of an ultrasound image. In such a case, the amount of calculation can be decreased to a large extent. In such a case, until an optimal attenuation rate is set next time, the value of the optimal attenuation rate that is set late may be used.

The statistical dispersion may be calculated on a target area for each sound ray or on an area with the reception depth being a predetermined value or more. The input unit 35 may be configured to receive a setting of such an area.

The input unit 35 may be configured to receive an input of a setting change of the set values $\alpha_1$, $\alpha_2$, and $\alpha_3$ of the attenuation rate candidate values.

As a quantity giving a statistical dispersion, for example, any one of a standard deviation, a difference between a maximum value and a minimum value of feature in a population, a half-value width of the distribution of feature may be applied. As a quantity giving a statistical dispersion, a reciprocal of the dispersion may be applied. However, in such a case, it is apparent that the quadratic function thereof is convex upward, and an attenuation rate candidate value corresponding to the extreme value thereof is set as an optimal attenuation rate.

The optimal attenuation rate setting unit 333c may calculate each statistical dispersion of a plurality of kinds of corrected feature and set an attenuation rate candidate value corresponding to an extreme value of a quadratic function generated based on the statistical dispersion as an optimal partial attenuation rate.

Furthermore, an ultrasound miniature probe having a small diameter having no optical system may be applied as an ultrasound probe. The ultrasound miniature probe, generally, is inserted into a bile duct, a biliary tract, a pancreatic duct, trachea, a bronchial tube, urethra, or a urinary duct and is used when peripheral organs thereof (pancreas, lung, prostate, urinary bladder, a lymph node, and the like) are observed.

As the ultrasound probe, an external-type ultrasound probe emitting an ultrasound wave from the surface of a subject may be employed. The external-type ultrasound probe, generally, is used when organs in the abdomen (liver, gallbladder, or urinary bladder), mamma (particularly, mammary gland), or thyroid gland is observed.

Furthermore, the ultrasound transducer may be a linear transducer, a radial transducer, or a convex transducer. In a case where the ultrasound transducer is a linear transducer, the scanning area thereof forms a rectangle (a rectangle or a square). On the other hand, in a case where the ultrasound transducer is a radial transducer or a convex transducer, the scanning area thereof forms a linear shape or a circular shape. The ultrasound endoscope may allow the ultrasound transducer to perform mechanical scanning or to perform electronic scanning by arranging a plurality of elements as ultrasound transducers in an array pattern and electronically performing switching among the elements relating to transmission/reception or applying a delay to the transmission/reception of each element.

In this way, the present invention may include various embodiments in a range not departing from the technical idea described in the claims.

## Industrial Applicability

As described above, an ultrasound observation apparatus, a method for operating the ultrasound observation apparatus, and a program for operating the ultrasound observation apparatus are useful for acquiring attenuation characteristics of ultrasound waves suitable for an observation target through simple calculation and performing an observation using the attenuation characteristics.

## Reference Signs List

| | |
|---|---|
| 1 | ULTRASOUND OBSERVATION SYSTEM |
| 2 | ULTRASOUND ENDOSCOPE |
| 3 | ULTRASOUND OBSERVATION APPARATUS |
| 4 | DISPLAY DEVICE |
| 21 | ULTRASOUND TRANSDUCER |
| 31 | TRANSMITTING AND RECEIVING UNIT |
| 32 | SIGNAL PROCESSING UNIT |
| 33 | COMPUTING UNIT |
| 34 | IMAGE PROCESSING UNIT |
| 35 | INPUT UNIT |
| 36 | CONTROL UNIT |
| 37 | STORAGE UNIT |

| | |
|---|---|
| 201 | FEATURE IMAGE |
| 202 | SUPERIMPOSED IMAGE DISPLAY SECTION |
| 203 | INFORMATION DISPLAY SECTION |
| 331 | AMPLIFICATION CORRECTING UNIT |
| 332 | FREQUENCY ANALYZING UNIT |
| 333 | FEATURE CALCULATING UNIT |
| 333a | APPROXIMATION UNIT |
| 333b | ATTENUATION CORRECTING UNIT |
| 333c | OPTIMAL ATTENUATION RATE SETTING UNIT |
| 341 | B-MODE IMAGE DATA GENERATING UNIT |
| 342 | FEATURE IMAGE DATA GENERATING UNIT |
| 371 | FEATURE INFORMATION STORING UNIT |
| $C_1$ | FREQUENCY SPECTRUM |

## Claims

1. An ultrasound observation apparatus comprising:

   a frequency analyzing unit configured to calculate a plurality of frequency spectra by analyzing a frequency of a signal generated based on an echo signal acquired by converting an ultrasound echo into an electric signal, the ultrasound echo being an ultrasound wave transmitted to an observation target and reflected from the observation target;
   a feature calculating unit configured to:

      calculate features of the plurality of frequency spectra;
      perform an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra;
      calculate a statistical dispersion of the corrected features for each of the at least three attenuation rate candidate values;
      generate a quadratic function based on the statistical dispersion; and
      set one of the at least three attenuation rate candidate values which gives a minimum statistical dispersion in the quadratic function, as an optimal attenuation rate; and

   a feature image data generating unit configured to generate feature image data for displaying the corrected features based on the optimal attenuation rate in association with visual information together with an ultrasound image generated from the echo signal.

2. The ultrasound observation apparatus according to claim 1, wherein
   the feature calculating unit is configured to set the optimal attenuation rate by using data of a dynamic range wider than a dynamic range of data used by the feature image data generating unit.

3. The ultrasound observation apparatus according to claim 1 or 2, wherein
   the feature calculating unit is configured to approximate each of the plurality of frequency spectra by an n-th order expression (n is a positive integer) to calculate the features.

4. The ultrasound observation apparatus according to claim 3, wherein
   the feature calculating unit is configured to:

   approximate a predetermined frequency band of each of the plurality of frequency spectra by a linear expression;
   calculate, as the features, one or more of an intercept of the linear expression, a slope of the linear expression, and a mid-band fit that is a value of the linear expression in an intermediate frequency of the predetermined frequency band, the features including one of the slope and the mid-band fit; and
   set the optimal attenuation rate based on one of the slope and the mid-band fit.

5. The ultrasound observation apparatus according to claim 4, wherein

the feature calculating unit is configured to:

set the optimal attenuation rate based on the slope if the slope is calculated as the features; and
set the optimal attenuation rate based on the mid-band fit if the mid-band fit is calculated as the features.

**6.** The ultrasound observation apparatus according to any one of claims 1 to 5, wherein the feature calculating unit is configured to set the optimal attenuation rate for all frames of the ultrasound image.

**7.** The ultrasound observation apparatus according to any one of claims 1 to 5, wherein the feature calculating unit is configured to:

set the optimal attenuation rate for every predetermined number of frames larger than one frame of the ultrasound image; and
calculate the features of each of the plurality of frequency spectra for a frame for which the optimal attenuation rate is not set, by using the optimal attenuation rate that is set last before the frame.

**8.** The ultrasound observation apparatus according to any one of claims 1 to 5, wherein the feature calculating unit is configured to:

calculate optimal attenuation rate correspondence values corresponding to the optimal attenuation rate for all frames of the ultrasound image; and
set the optimal attenuation rate based on the optimal attenuation rate correspondence values calculated for a predetermined number of frames larger than one frame.

**9.** The ultrasound observation apparatus according to any one of claims 1 to 8, wherein the feature image data contains information on the optimal attenuation rate.

**10.** The ultrasound observation apparatus according to any one of claims 1 to 9, further comprising a display unit configured to display a feature image corresponding to the feature image data.

**11.** The ultrasound observation apparatus according to any one of claims 1 to 10, further comprising an input unit configured to receive an input for setting a target area for which the plurality of frequency spectra is calculated by the frequency analyzing unit, wherein the frequency analyzing unit is configured to calculate the plurality of frequency spectra based on the ultrasound echo reflected from the target area.

**12.** A method for operating an ultrasound observation apparatus, the method comprising:

a frequency analyzing step of calculating a plurality of frequency spectra by analyzing a frequency of a signal generated based on an echo signal acquired by converting an ultrasound echo into an electric signal, the ultrasound echo being an ultrasound wave transmitted to an observation target and reflected from the observation target;
a feature calculating step of, by a feature calculating unit:

calculating features of the plurality of frequency spectra;
performing an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra;
calculating a statistical dispersion of the corrected features for each of the at least three attenuation rate candidate values;
generating a quadratic function based on the statistical dispersion; and
setting one of the at least three attenuation rate candidate values which gives a minimum statistical dispersion in the quadratic function, as an optimal attenuation rate; and

a feature image data generating step of, by a feature image data generating unit, generating feature image data for displaying the corrected features based on the optimal attenuation rate in association with visual information together with an ultrasound image generated from the echo signal.

**13.** A program for operating an ultrasound observation apparatus, the program causing the ultrasound observation apparatus to execute:

a frequency analyzing step of calculating a plurality of frequency spectra by analyzing a frequency of a signal generated based on an echo signal acquired by converting an ultrasound echo into an electric signal, the ultrasound echo being an ultrasound wave transmitted to an observation target and reflected from the observation target;
a feature calculating step of, by a feature calculating unit:

calculating features of the plurality of frequency spectra;
performing an attenuation correction for excluding an influence of attenuation of the ultrasound wave, on the features of the plurality of frequency spectra for each of at least three attenuation rate candidate values giving different attenuation characteristics in propagating the ultrasound wave through the observation target, thereby calculating corrected features of the plurality of frequency spectra;
calculating a statistical dispersion of the corrected features for each of the at least three attenuation rate candidate values;
generating a quadratic function based on the statistical dispersion; and
setting one of the at least three attenuation rate candidate values which gives a minimum statistical dispersion in the quadratic function, as an optimal attenuation rate; and

a feature image data generating step of, by a feature image data generating unit, generating feature image data for displaying the corrected features based on the optimal attenuation rate in association with visual information together with an ultrasound image generated from the echo signal.

FIG.1

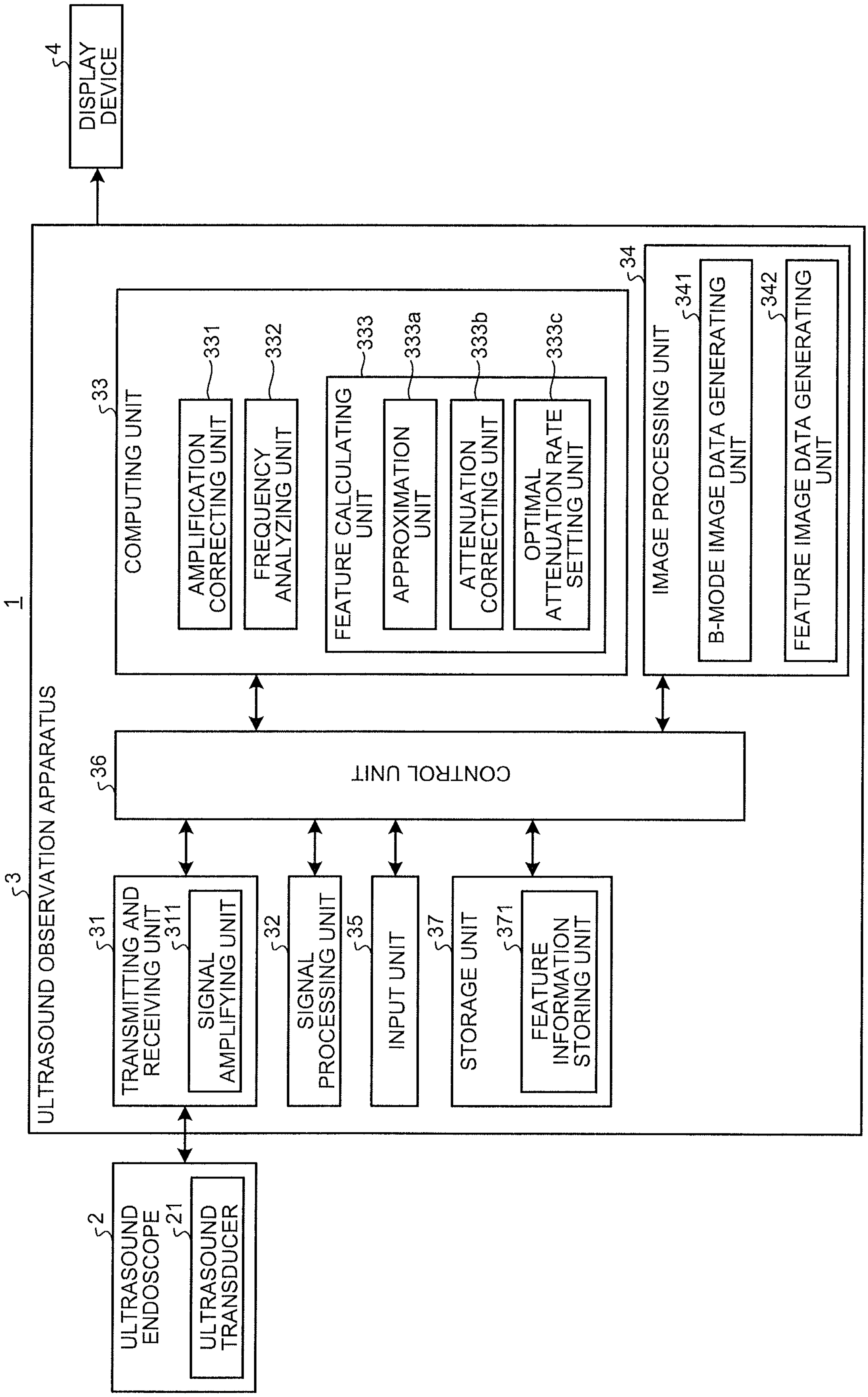
1
ULTRASOUND OBSERVATION APPARATUS
3
DISPLAY DEVICE
4
COMPUTING UNIT
33
AMPLIFICATION CORRECTING UNIT
331
FREQUENCY ANALYZING UNIT
332
FEATURE CALCULATING UNIT
333
APPROXIMATION UNIT
333a
ATTENUATION CORRECTING UNIT
333b
OPTIMAL ATTENUATION RATE SETTING UNIT
333c
IMAGE PROCESSING UNIT
34
B-MODE IMAGE DATA GENERATING UNIT
341
FEATURE IMAGE DATA GENERATING UNIT
342
CONTROL UNIT
36
TRANSMITTING AND RECEIVING UNIT
31
SIGNAL AMPLIFYING UNIT
311
SIGNAL PROCESSING UNIT
32
INPUT UNIT
35
STORAGE UNIT
37
FEATURE INFORMATION STORING UNIT
371
ULTRASOUND ENDOSCOPE
2
ULTRASOUND TRANSDUCER
21

# FIG.2

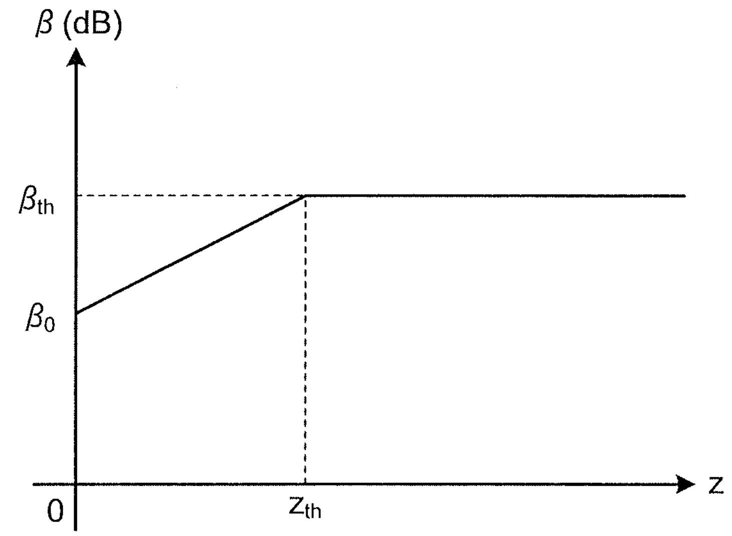


# FIG.3

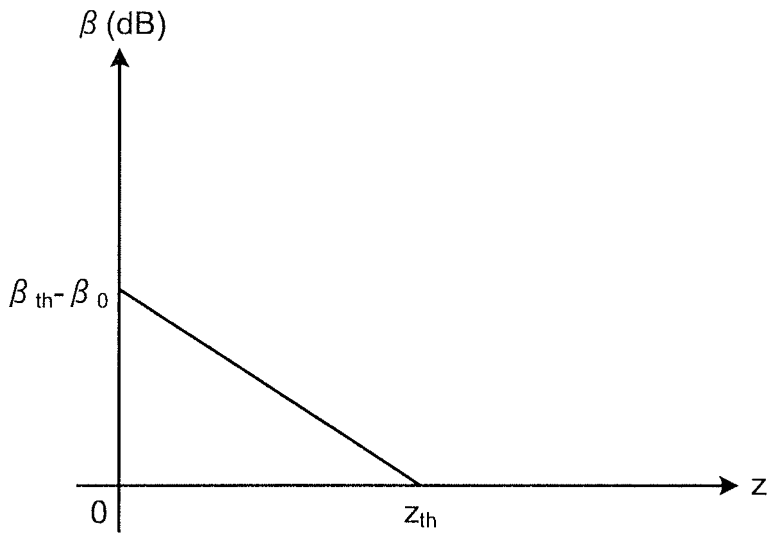

FIG.4

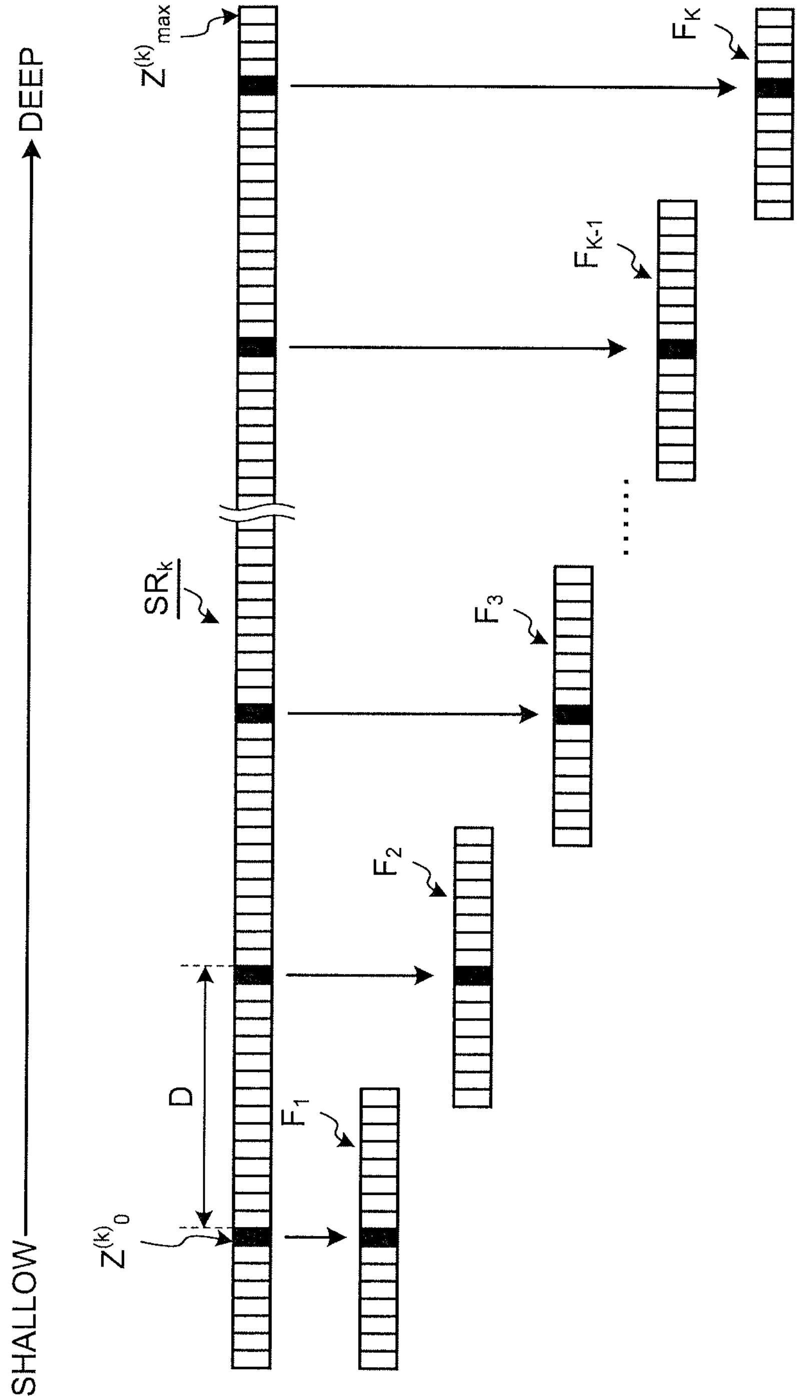

FIG.5

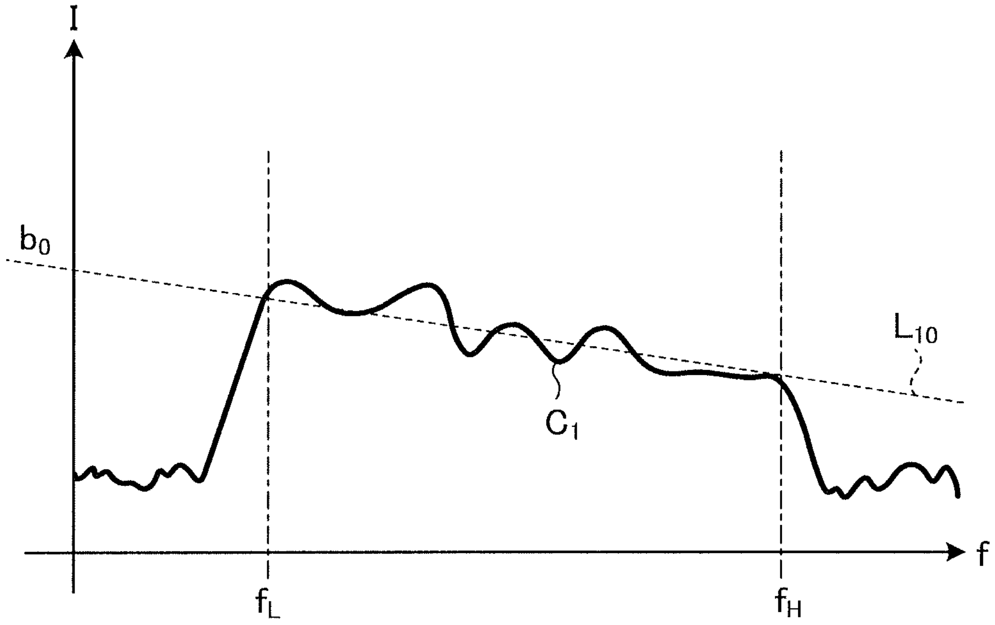


FIG.6

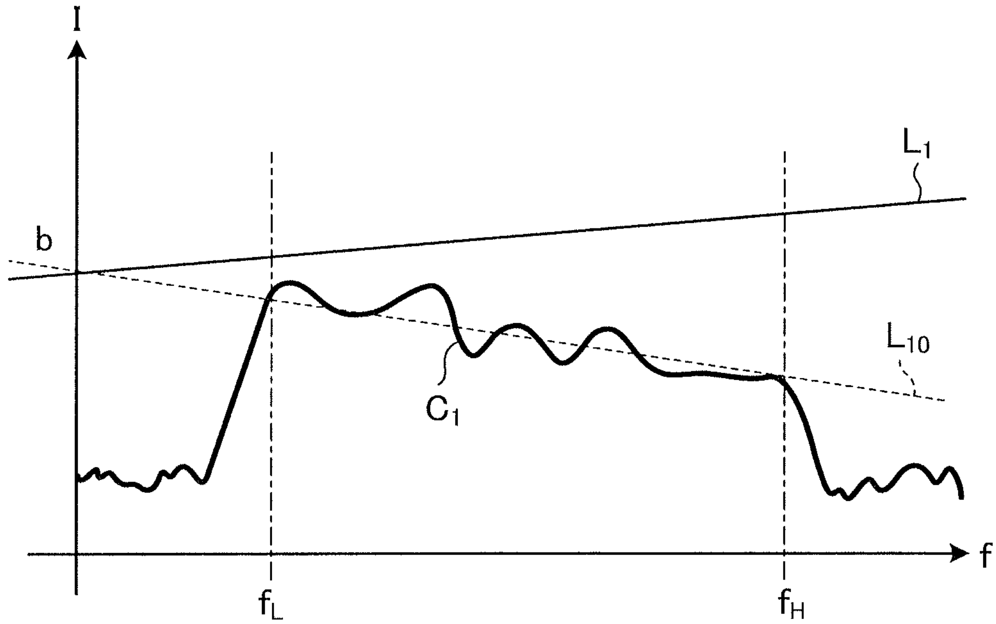

FIG.7

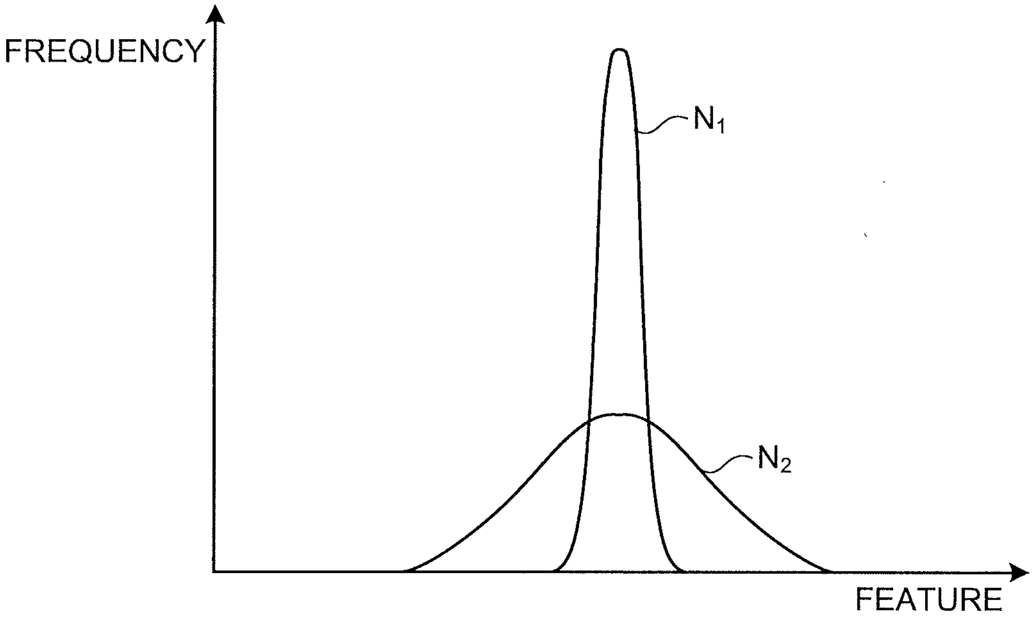
FREQUENCY
N1
N2
FEATURE

FIG.8

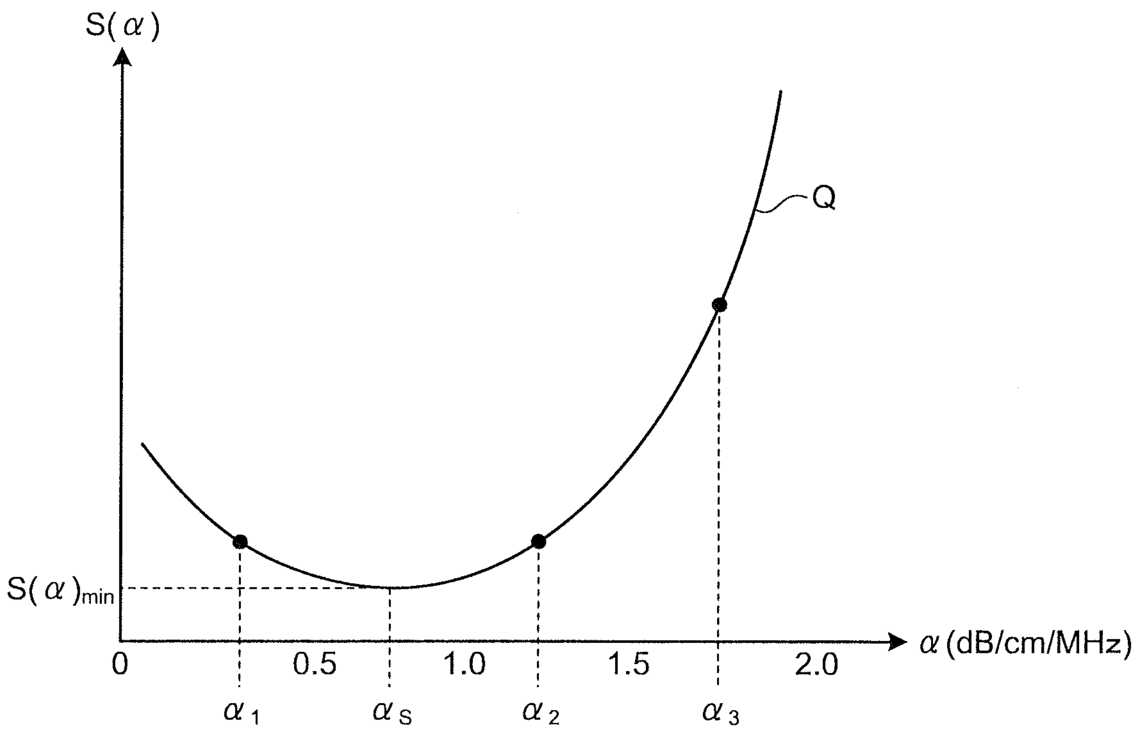
S(α)
Q
S(α)min
α (dB/cm/MHz)
0
0.5
1.0
1.5
2.0
α1
αS
α2
α3

FIG.9

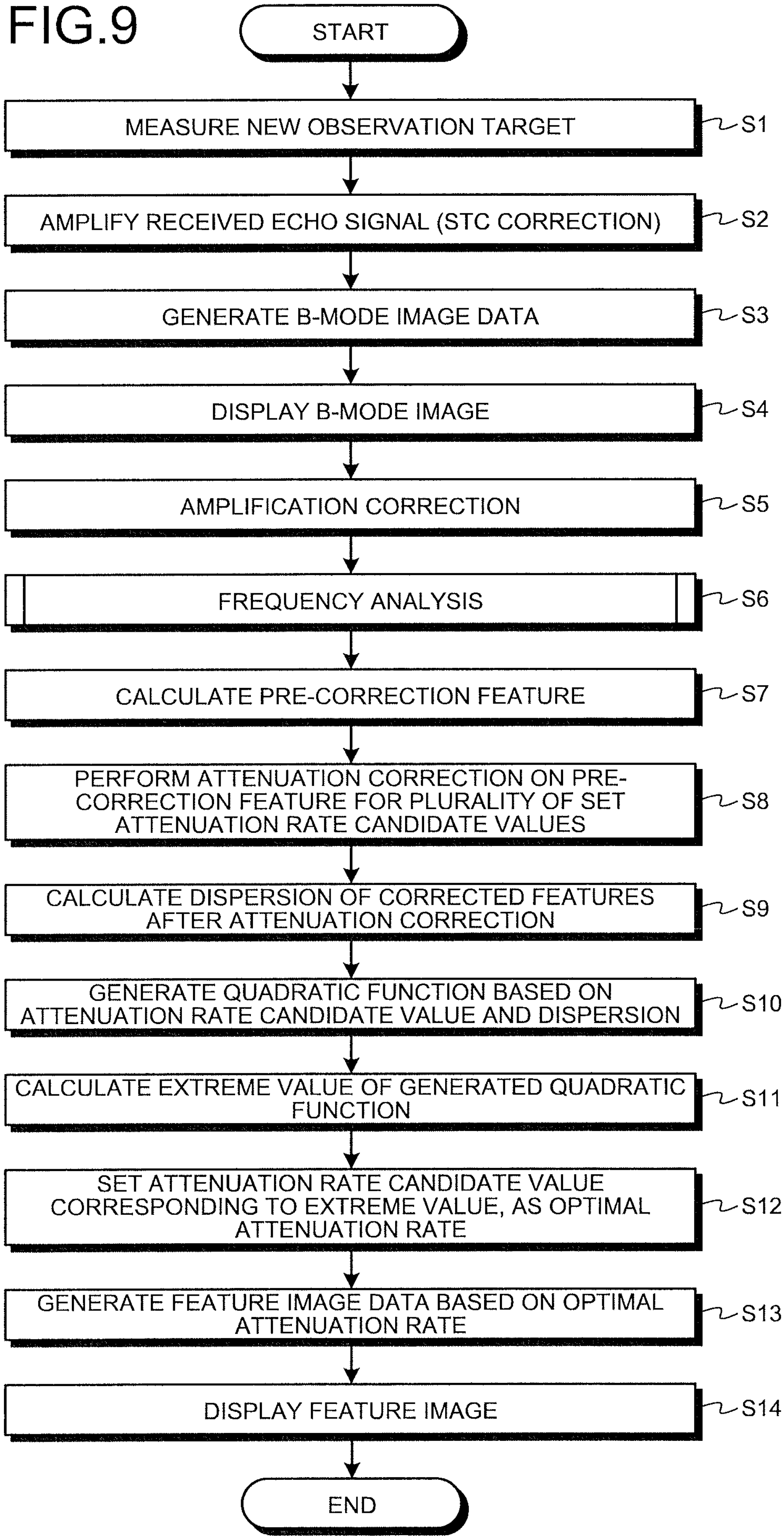
START
MEASURE NEW OBSERVATION TARGET
S1
AMPLIFY RECEIVED ECHO SIGNAL (STC CORRECTION)
S2
GENERATE B-MODE IMAGE DATA
S3
DISPLAY B-MODE IMAGE
S4
AMPLIFICATION CORRECTION
S5
FREQUENCY ANALYSIS
S6
CALCULATE PRE-CORRECTION FEATURE
S7
PERFORM ATTENUATION CORRECTION ON PRE-CORRECTION FEATURE FOR PLURALITY OF SET ATTENUATION RATE CANDIDATE VALUES
S8
CALCULATE DISPERSION OF CORRECTED FEATURES AFTER ATTENUATION CORRECTION
S9
GENERATE QUADRATIC FUNCTION BASED ON ATTENUATION RATE CANDIDATE VALUE AND DISPERSION
S10
CALCULATE EXTREME VALUE OF GENERATED QUADRATIC FUNCTION
S11
SET ATTENUATION RATE CANDIDATE VALUE CORRESPONDING TO EXTREME VALUE, AS OPTIMAL ATTENUATION RATE
S12
GENERATE FEATURE IMAGE DATA BASED ON OPTIMAL ATTENUATION RATE
S13
DISPLAY FEATURE IMAGE
S14
END

FIG.10

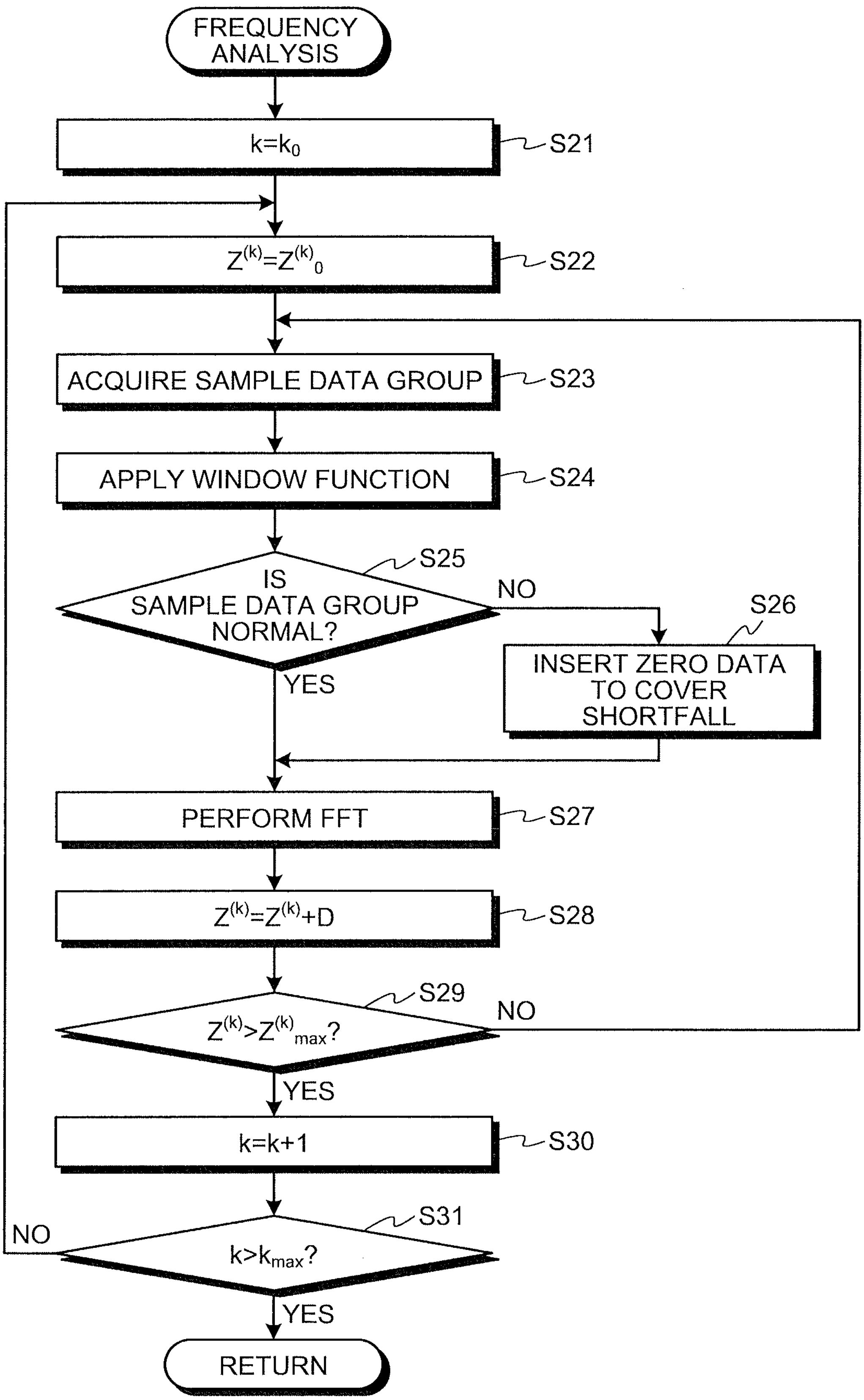
FREQUENCY ANALYSIS
k=k0
S21
Z(k)=Z(k)0
S22
ACQUIRE SAMPLE DATA GROUP
S23
APPLY WINDOW FUNCTION
S24
IS SAMPLE DATA GROUP NORMAL?
S25
NO
YES
INSERT ZERO DATA TO COVER SHORTFALL
S26
PERFORM FFT
S27
Z(k)=Z(k)+D
S28
Z(k)>Z(k)max?
S29
NO
YES
k=k+1
S30
k>kmax?
S31
NO
YES
RETURN

FIG.11

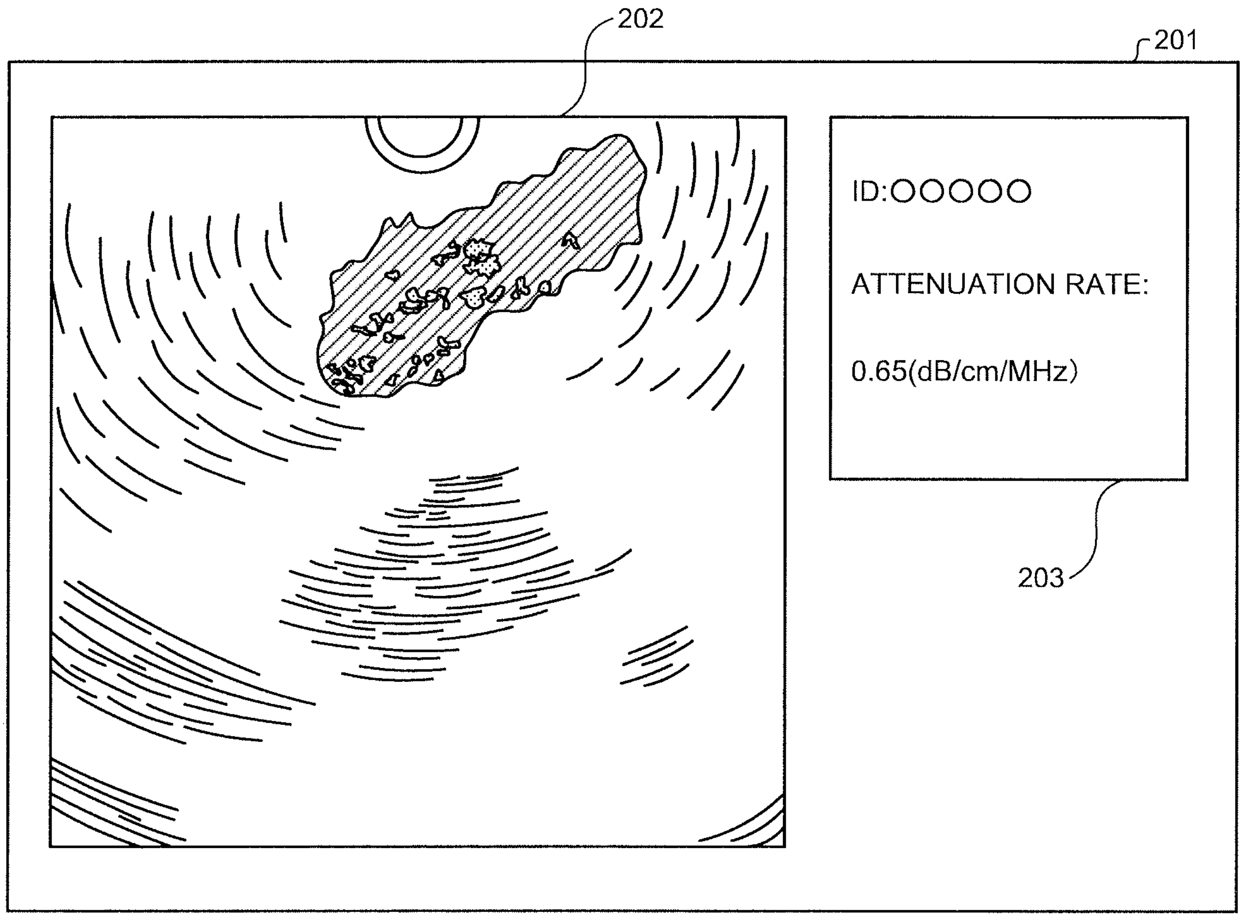

## INTERNATIONAL SEARCH REPORT

International application No. PCT/JP2015/083938

A. CLASSIFICATION OF SUBJECT MATTER
*A61B8/14*(2006.01)i, *A61B8/12*(2006.01)i

According to International Patent Classification (IPC) or to both national classification and IPC

B. FIELDS SEARCHED

Minimum documentation searched (classification system followed by classification symbols)
A61B8/14, A61B8/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2012/063929 A1 (Olympus Medical Systems Corp.), 18 May 2012 (18.05.2012), abstract; paragraphs [0029] to [0041], [0068] to [0070]; fig. 2, 3, 7 to 10 & US 2013/0030296 A1 fig. 2, 3, 7 to 10; paragraphs [0031] to [0044], [0071] to [0073] & CN 103153195 A | 1-13 |
| A | JP 62-117536 A (Shimadzu Corp.), 29 May 1987 (29.05.1987), page 2, lower right column, line 15 to page 3, upper right column, line 11 (Family: none) | 1-13 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February 2016 (19.02.16) | 01 March 2016 (01.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008545123 T